# EUROPÄISCHE PATENTSCHRIFT

(11) **EP 2 932 959 B1**
(45) Veröffentlichungstag und Bekanntmachung des Hinweises auf die Patenterteilung: **13.09.2017**
(21) Anmeldenummer: 15161851.9
(22) Anmeldetag: 31.03.2015
(51) Int. Cl.: A61K 8/44, A61K 8/46, A61Q 19/10, A61K 8/92, A61K 8/06, C11D 1/94

(54) **TENSIDZUSAMMENSETZUNGEN UND HOCH ÖLHALTIGE FORMULIERUNGEN ENTHALTEND DIESE**
SURFACTANT COMPOSITIONS AND HIGHLY OLEAGINOUS FORMULATIONS CONTAINING SAME
COMPOSITIONS DE TENSIO-ACTIFS ET FORMULES À FORTE TENEUR EN HUILE LES COMPRENANT

(30) Priorität: 17.04.2014 DE 102014207386
(43) Veröffentlichungstag der Anmeldung: 21.10.2015
(73) Patentinhaber: Evonik Degussa GmbH, 45128 Essen (DE)
(72) Erfinder: Kleinen, Jochen, 52525 Heinsberg (DE); Kortemeier, Uta, 45128 Essen (DE); Hartung, Christian, 45133 Essen (DE); Venzmer, Joachim, 45239 Essen (DE)

(56) Entgegenhaltungen:
- EP-A2- 0 232 153
- WO-A1-2008/118381
- US-A- 4 243 549
- US-A1- 2002 169 093
- US-A1- 2003 008 792
- US-A1- 2003 153 631
- US-A1- 2005 227 892
- US-A1- 2012 066 839

## Beschreibung

### Gebiet der Erfindung

Gegenstand der Erfindung sind Zusammensetzungen, die zur Herstellung strukturierter Formulierungen genutzt werden können, strukturierte Formulierungen enthaltend diese Zusammensetzungen sowie Verfahren zur Herstellung von strukturierten Formulierungen.

### Stand der Technik

Strukturierte tensidhaltige Formulierungen sind flüssigkristalline Zusammensetzungen, in denen die Tenside in Form von planaren und/oder sphärolithischen lamellaren Phasen vorliegen. Üblicherweise liegen die Tensid-Phasen als Sphärolithe, d.h. als lamellare Tröpfchen in der wässrigen Phase dispergiert vor. Solche Sphärolithe bestehen aus einer zwiebelartigen Konfiguration von konzentrischen Doppelschichten von Tensid-Molekülen, zwischen denen Wasser oder Elektrolytlösung eingefangen ist. Strukturierte Tensidzusammensetzungen sind typischerweise pumpbare, nicht-Newtonsche Zusammensetzungen, welche wasserunlösliche Teilchen, wie z.B. Öltröpfchen, aufgrund der Anwesenheit dieser lamellaren Tensid-Phasen zu suspendieren vermögen.

Solch strukturierte Formulierungen finden zum Beispiel in kosmetischen Reinigungsformulierungen wie Shampoo, Duschgel, Seife, Gesichtsreiniger, Schaumbad und Körperpflegeformulierungen wie Lotionen, Cremes, Conditioner, Rasur-Produkte und Babypflegeformulierungen Verwendung.

Eine herausragende Eigenschaft solcher strukturierten Formulierungen ist deren Fähigkeit, in ihnen enthaltene Substanzen auf einer Oberfläche bei Zugabe von Wasser effizient zu depositionieren.

Die WO2008118381 offenbart strukturierte oberflächenaktive Zusammensetzungen, umfassend, bezogen auf 100 Gewichtsteile der Zusammensetzung,
(I) von größer als 0 bis etwa 15 Gewichtsteile eines Alkylethersulfat-Tensids,
(II) mehr als 0 bis etwa 15 Gewichtsteile eines Alkyl-Sulfat-Tensids,
(III) mehr als 0 bis etwa 8 Gewichtsteile eines Alkanolamid-Tensids,
(IV) 0 bis etwa 10 Gewichtsteile eines amphoteren Tensids,
   mit der Maßgabe, dass die Gesamtmenge der Komponenten (I), (II), (III) und (IV) größer als oder gleich 5 Gewichtsteile ist,
(V) eine Menge eines Elektrolyten, die bewirkt, in Kombination mit den Komponenten (I), (II), (III) und (IV) eine strukturierte Tensidzusammensetzung mit einem opaken visuellen Erscheinungsbild und einer Fließgrenze von mehr als 0 Pascal zu erzeugen, und
(VI) Wasser.

Nachteil der im Stand der Technik beschriebenen Formulierungen ist es, dass diese kein besonders gutes Schaumverhalten aufweisen.
Ebenso nachteilig ist der notwendige Gehalt an Alkylethersulfaten in den Formulierungen des Standes der Technik.
Ein weiterer Nachteil der strukturierten Formulierungen des Standes der Technik ist es, dass das Verhältnis von Tensiden zu enthaltenen Ölen hoch ist.

Aufgabe der Erfindung war es, gut schäumende, strukturierte Formulierungen mit einem hohen Ölanteil, wie beispielsweise 20-60 Gew.-% bezogen auf die Gesamtformulierung, bereitzustellen.

### Beschreibung der Erfindung

Unerwarteter Weise wurde gefunden, dass sich mit Hilfe der Zusammensetzung des Anspruches 1 gut schäumende, strukturierte Formulierungen mit einem hohen Ölanteil herstellen lassen. Da Dialkylsulfosuccinate wie z.B. in der US3650964 beschrieben generell als schlecht schäumende Tenside bekannt sind und insbesondere auch hochölhaltige Formulierungen, die zum Beispiel mehr als 10 Gew.-% Öl enthalten, normalerweise nur geringen Schaum bei kosmetischen Anwendungen zeigen, ist die vorliegende Erfindung sehr überraschend.

Gegenstand der vorliegenden Erfindung sind daher Zusammensetzungen wie in Anspruch 1 beschrieben.

Ein weiterer Gegenstand der vorliegenden Erfindung sind strukturierte Formulierungen enthaltend die erfindungsgemäßen Zusammensetzungen.

Noch ein Gegenstand der vorliegenden Erfindung sind Verfahren zur Herstellung der erfindungsgemäßen, strukturierten Formulierungen.

Ein Vorteil der vorliegenden Erfindung ist es, dass in die erfindungsgemäßen Formulierungen hohe Mengen an Öl eingearbeitet werden können.
Ein weiterer Vorteil der vorliegenden Erfindung ist es, dass die erfindungsgemäßen Formulierungen sulfatfrei, insbesondere ethersulfatfrei erstellt werden können.
Ein weiterer Vorteil der vorliegenden Erfindung ist es, dass die erfindungsgemäßen Formulierungen polyetherfrei erstellt werden können.
Noch ein Vorteil der vorliegenden Erfindung ist es, dass die erfindungsgemäßen Formulierungen bezogen auf enthaltene Menge an Tensid sehr viel Öl stabil enthalten können.

Ein weiterer Vorteil ist es, dass die Formulierungen sehr mild bzgl. Haut- und Augenreizungspotential formuliert werden können.
Ein weiterer Vorteil der vorliegenden Erfindung ist es, dass die erfindungsgemäßen Formulierungen lagerstabil sind, sowohl bei erhöhten, als auch bei erniedrigten Temperaturen als auch bei Temperaturschwankungen.

Noch ein Vorteil der vorliegenden Erfindung ist es, dass die erfindungsgemäßen Formulierungen Duft-, Wirk- und Pflegestoffe stabil enthalten können und diese in einem hohen Maß zu depositionieren vermögen. Das hat den Vorteil, dass z.B. der Duftstoff auf der Oberfläche gleichmäßig verteilt und langanhaltend freigegeben wird. Ein weiterer Vorteil der vorliegenden Erfindung ist es, dass die erfindungsgemäßen Formulierungen teilweise kalt und/oder ohne großen Energieeintrag herstellbar sind. Noch ein Vorteil der vorliegenden Erfindung ist es, dass die erfindungsgemäßen Formulierungen ohne Fettsäuren und Fettalkohole formuliert werden können. Ein weiterer Vorteil der vorliegenden Erfindung ist es, dass die erfindungsgemäßen Formulierungen ohne die Zugabe von polymeren rheologischen Additiven eine Fließgrenze aufweisen können.

Ein weiterer Vorteil der vorliegenden Erfindung ist es, dass die erfindungsgemäßen Formulierungen trotz des hohen Anteils an Öl stark schäumen.

Ein weiterer Vorteil der vorliegenden Erfindung ist es, dass die erfindungsgemäßen Formulierungen sich gut abspülen lassen.

Noch ein Vorteil der vorliegenden Erfindung ist es, dass das Hautgefühl während und nach dem Waschen verbessert wird.

Ein weiterer Vorteil der vorliegenden Erfindung ist die verbesserte Waschbeständigkeit oxidativer Haarfarben.

Ein weiterer Vorteil der vorliegenden Erfindung ist es, dass die erfindungsgemäßen Formulierungen bei Anwendung auf dem Haar zu verbessertem Glanz und zu verbesserter Konditionierung der Haare führen.

Noch ein Vorteil der vorliegenden Erfindung ist die spezielle cremige und reichhaltige Textur der Formulierungen, die erhalten werden können. Zudem ist diese Textur ohne die Zugabe von größeren Mengen Salz zu erhalten.

Die erfindungsgemäße Zusammensetzung enthält
A) 20 Gewichtsteile bis 67 Gewichtsteile, bevorzugt 25 Gewichtsteile bis 57 Gewichtsteile, besonders bevorzugt 33 Gewichtsteile bis 47 Gewichtsteile, mindestens einer Substanz ausgewählt aus Dialkylsulfosuccinaten und Dialkylmethylsulfosuccinaten,
B) 15 Gewichtsteile bis 40 Gewichtsteile, bevorzugt 20 Gewichtsteile bis 32 Gewichtsteile, besonders bevorzugt 23 Gewichtsteile bis 28 Gewichtsteile, mindestens eines amphoteren Tensids, und
C) 15 Gewichtsteile bis 40 Gewichtsteile, bevorzugt 20 Gewichtsteile bis 32 Gewichtsteile, besonders bevorzugt 23 Gewichtsteile bis 28 Gewichtsteile, mindestens eines anionischen Tensids.

Unter dem Begriff "Tensid" im Zusammenhang mit der vorliegenden Erfindung werden organische Substanzen mit grenzflächenaktiven Eigenschaften verstanden, die die Fähigkeit besitzen, die Oberflächenspannung von Wasser bei 20°C und bei einer Konzentration von 0,5 Gew.-% bezogen auf die Gesamtzusammensetzung auf unter 45 mN/m zu verringern. Die Oberflächenspannung wird hier durch die Ringmethode nach DuNoüy bei 25°C bestimmt.

Der Begriff "Alkyl-" im Zusammenhang mit der vorliegenden Erfindung umfasst Alkylreste, die gegebenenfalls ungesättigt sein können.

Unter dem Begriff "amphoteres Tensid" wird im Zusammenhang mit der vorliegenden Erfindung ein zwitterionisches Tensid verstanden, bei welchem bei einem pH-Wert von 7 und 20 °C mindestens 90 mol-% der Moleküle sowohl jeweils mindestens eine negativ und positiv geladene Gruppe aufweisen. Bevorzugt weisen sie einen isolelektrischen Punkt von pH_{IEP}=2-12 bei 25°C auf, wobei in einer wässrigen 10 millimolaren Kaliumchloridlösung als Hintergrundelektrolyt gemessen wird.

Unter dem Begriff "anionisches Tensid" wird im Zusammenhang mit der vorliegenden Erfindung ein Tensid verstanden, bei welchem bei einem pH-Wert von 7 und 20 °C mindestens 90 mol-% der Moleküle mindestens eine negativ geladene Gruppe aufweisen. Bevorzugt weisen sie keinen isolelektrischen Punkt von pH_{IEP}=2-12 bei 25°C auf, wobei in einer wässrigen 10 millimolaren Kaliumchloridlösung als Hintergrundelektrolyt gemessen wird. Dialkylsulfosuccinate und Dialkylmethylsulfosuccinate sind dabei ausgeschlossen.

Alle angegebenen Prozent (%) sind, wenn nicht anders angegeben, Massenprozent. Werden in bevorzugten Ausführungsformen der vorliegenden Erfindung eine oder mehrere der enthaltenen Komponenten auf mindestens eine definierte Substanz eingeschränkt, so ist dies so zu verstehen, dass keine weitere Substanz der Komponente außer der genannten in der Zusammensetzung oder in der Formulierung enthalten ist.

Bevorzugt in der erfindungsgemäßen Zusammensetzung enthaltene Dialkylsulfosuccinate und/oder Dialkylmethylsulfosuccinate sind ausgewählt aus der Gruppe bestehend aus Dialkylsulfosuccinaten und/oder Dialkylmethylsulfosuccinaten, bei denen die Alkylreste jeweils 4 bis 24, bevorzugt 6 bis 18, besonders bevorzugt 6 bis 14, Kohlenstoffatome aufweisen. Die Alkylreste können linear oder verzweigt sein, wobei verzweigte bevorzugt sind. Es können unterschiedliche oder identische Alkylreste in einem Molekül Dialkylsulfosuccinat und/oder Dialkylmethylsulfosuccinat enthalten sein, wobei identische bevorzugt sind.

Als Gegenion zur Sulfonsäuregruppe können bevorzugt Alkalimetallkationen, Erdalkalikationen oder Ammonium-Ionen, insbesondere Natrium eingesetzt werden. Besonders bevorzugt enthaltene Dialkylsulfosuccinate sind ausgewählt aus der Gruppe bestehend aus Diethylhexyl Sodium Sulfosuccinate, Dinonyl Sodium Sulfosuccinate, Diisononyl Sodium Sulfosuccinate, Dioctyl Sodium Sulfosuccinate, Diheptyl Sodium Sulfosuccinate, Dihexyl Sodium Sulfosuccinate, Dineopentyl Sodium Sulfosuccinate, Diisoamyl Sodium Sulfosuccinate, Dipentyl Sodium Sulfosuccinate, Diamyl Sodium Sulfosuccinate, Dibutyl Sodium Sulfosuccinate, Diisobutyl Sodium Sulfosuccinate, Dicapryl Sodium Sulfosuccinate, Didecyl Sodium Sulfosuccinate, Diundecyl Sodium Sulfosuccinate, Dilauryl Sodium Sulfosuccinate, Dicocoyl Sodium Sulfosuccinate, Ditridecyl Sodium Sulfosuccinate, Dipropylheptyl Sodium Sulfosuccinate, Dicyclohexyl Sodium Sulfosuccinate, Ammonium Diethylhexyl Sulfosuccinate, Ammonium Dinonyl Sulfosuccinate, Ammonium Diisononyl Sulfosuccinate, Ammonium Dioctyl Sodium Sulfosuccinate, Ammonium Diheptyl Sulfosuccinate, Ammonium Dihexyl Sulfosuccinate, Ammonium Dineopentyl Sulfosuccinate, Ammonium Diisoamyl Sulfosuccinate, Ammonium Dipentyl Sulfosuccinate, Ammonium Diamyl Sulfosuccinate, Ammonium Dibutyl Sulfosuccinate, Ammonium Diisobutyl Sulfosuccinate, Ammonium Dicapryl Sulfosuccinate, Ammonium Didecyl Sulfosuccinate, Ammonium Diundecyl Sulfosuccinate, Ammonium Dilauryl Sulfosuccinate, Ammonium Dicocoyl Sulfosuccinate, Ammonium Ditridecyl Sulfosuccinate, Ammonium Dipropylheptyl Sulfosuccinate, Ammonium Dicyclohexyl Sulfosuccinate, Diethylhexyl Potassium Sulfosuccinate, Dinonyl Potassium Sulfosuccinate, Diisononyl Potassium Sulfosuccinate, Dioctyl Potassium Sulfosuccinate, Diheptyl Potassium Sulfosuccinate, Dihexyl Potassium Sulfosuccinate, Dineopentyl Potassium Sulfosuccinate, Diisoamyl Potassium Sulfosuccinate, Dipentyl Potassium Sulfosuccinate, Diamyl Potassium Sulfosuccinate, Dibutyl Potassium Sulfosuccinate, Diisobutyl Potassium Sulfosuccinate, Dicapryl Potassium Sulfosuccinate, Didecyl Potassium Sulfosuccinate, Diundecyl Potassium Sulfosuccinate, Dilauryl Potassium Sulfosuccinate, Dicocoyl Potassium Sulfosuccinate, Ditridecyl Potassium Sulfosuccinate, Dipropylheptyl Potassium Sulfosuccinate, Dicyclohexyl Potassium Sulfosuccinate, Diethylhexyl Sodium Methylsulfosuccinate, Dinonyl Sodium Methylsulfosuccinate, Diisononyl Sodium Methylsulfosuccinate, Dioctyl Sodium Methylsulfosuccinate, Diheptyl Sodium Methylsulfosuccinate, Dihexyl Sodium Methylsulfosuccinate, Dineopentyl Sodium Methylsulfosuccinate, Diisoamyl Sodium Methylsulfosuccinate, Dipentyl Sodium Methylsulfosuccinate, Diamyl Sodium Methylsulfosuccinate, Dibutyl Sodium Methylsulfosuccinate, Diisobutyl Sodium Methylsulfosuccinate, Dicapryl Sodium Methylsulfosuccinate, Didecyl Sodium Methylsulfosuccinate, Diundecyl Sodium Methylsulfosuccinate, Dilauryl Sodium Methylsulfosuccinate, Dicocoyl Sodium Methylsulfosuccinate, Ditridecyl Sodium Methylsulfosuccinate, Dipropylheptyl Sodium Methylsulfosuccinate, Dicyclohexyl Sodium Methylsulfosuccinate, wobei Diethylhexyl Sodium Sulfosuccinate ganz besonders bevorzugt ist.

Bevorzugt in der erfindungsgemäßen Zusammensetzung enthaltene amphotere Tenside sind ausgewählt aus der Gruppe umfassend, bevorzugt bestehend aus Alkylbetaine, Alkylamidoalkylbetaine, Alkylamphoacetate, Alkylamphodiacetate, Alkylamphopropionate, Alkylamphodipropionate, Alkylsultaine, Alkylhydroxysultaine, Alkylaminoxide, Alkylamphoglycinate, Alkyliminodiacetate, Alkyliminodipropionate, Alkylamphopropylsulfonate, Alkylamphocarboxyglycinaten und Alkylamphocarboxypropionaten.

Besonders bevorzugt in der erfindungsgemäßen Zusammensetzung enthaltene amphotere Tenside sind ausgewählt aus der Gruppe umfassend, bevorzugt bestehend aus, C4- bis C24-, bevorzugt C6- bis C18-, besonders bevorzugt C8- bis C14-, Alkylbetainen und C4- bis C24-, bevorzugt C6- bis C18-, besonders bevorzugt C8- bis C14-, Alkylamidopropylbetainen. Die Alkylreste können linear oder verzweigt sein, wobei lineare bevorzugt sind. Besonders bevorzugt enthaltene Alkylbetaine oder Alkylamidopropylbetaine sind ausgewählt aus der Gruppe bestehend aus Lauryl Betaine, Coco-Betaine, Behenyl Betaine, Capryl/Capramidopropyl Betaine, Cetyl Betaine, Cocamidoethyl Betaine, Cocamidopropyl Betaine, Coco/Oleamidopropyl Betaine, Decyl Betaine, Dimer Dilinoleamidopropyl Dibetaine, Hydrogenated Tallow Betaine, Hydroxylauryl/Hydroxymyristyl Betaine, Isostearamidopropyl Betaine, Lauramidopropyl Betaine, Lauryl Betaine, Myristyl Betaine, Oleamidopropyl Betaine, Oleyl Betaine, Palmitamidopropyl Betaine, Ricinoleamidopropyl Betaine, Stearamidopropyl Betaine, Stearyl Betaine, Tallowamidopropyl Betaine, Tallow Betaine, Tallow Dihydroxyethyl Betaine, Undecylenamidopropyl Betaine, Sunfloweramidopropyl Betaine, Cetyl Betaine, Lauryl Betaine.

Besonders bevorzugt als amphotere Tenside erfindungsgemäß enthaltene Alkylamphoacetate, Alkylamphodiacetate, Alkylamphopropionate oder Alkylamphodipropionate sind ausgewählt aus der Gruppe bestehend aus Cocobetainamido Amphopropionate, DEA-Cocoamphodipropionate, Disodium Caproamphodiacetate, Disodium Caproamphodipropionate, Disodium Capryloamphodiacetate, Disodium Capryloamphodipropionate, Disodium Cocoamphodiacetate, Disodium Cocoamphodipropionate, Disodium Isostearoamphodiacetate, Disodium Isostearoamphodipropionate, Disodium Laureth-5 Carboxyamphodiacetate, Disodium Lauroamphodiacetate, Disodium Lauroamphodipropionate, Disodium Oleoamphodipropionate, Disodium PPG-2-Isodeceth-7 Carboxyamphodiacetate, Disodium Stearoamphodiacetate, Disodium Tallowamphodiacetate, Sodium Caproamphopropionate, Sodium Capryloamphoacetate, Sodium Capryloamphopropionate, Sodium Cocoamphoacetate, Sodium Cocoamphopropionate, Sodium Isostearoamphoacetate, Sodium Isostearoamphopropionate, Sodium Lauroamphoacetate, Sodium Lauroamphopropionate, Sodium Myristoamphoacetate, Sodium Oleoamphoacetate, Sodium Oleoamphopropionate, Sodium Stearoamphoacetate, Sodium Stearoamphopropionate, Sodium Tallamphopropionate, Sodium Tallowamphoacetate, Sodium Undecylenoamphoacetate und Sodium Undecylenoamphopropionate.

Besonders bevorzugt als amphotere Tenside erfindungsgemäß enthaltene Alkylsultaine oder Alkylhydroxysultaine sind ausgewählt aus der Gruppe bestehend aus Capryl Sultaine, Cocamidopropyl Hydroxysultaine, Coco-Hydroxysultaine, Coco-Sultaine, Erucamidopropyl Hydroxysultaine, Lauramidopropyl Hydroxysultaine, Lauryl Hydroxysultaine, Lauryl Sultaine, Myristamidopropyl Hydroxysultaine, Oleamidopropyl Hydroxysultaine, Tallowamidopropyl Hydroxysultaine.

Besonders bevorzugt als amphotere Tenside enthaltene Alkylaminoxide sind ausgewählt aus der Gruppe bestehend aus
Behenamine Oxide, Cocamidopropylamine Oxide, Cocamine Oxide, Decylamine Oxide, Decyltetradecylamine Oxide, Dihydroxyethyl C8-10 Alkoxypropylamine Oxide, Dihydroxyethyl C9-11 Alkoxypropylamine Oxide, Dihydroxyethyl C12-15 Alkoxypropylamine Oxide, Dihydroxyethyl Cocamine Oxide, Dihydroxyethyl Lauramine Oxide, Dihydroxyethyl Stearamine Oxide, Dihydroxyethyl Tallowamine Oxide, Hydrogenated Tallowamine Oxide, Isostearamidopropylamine Oxide, Lauramidopropylamine Oxide, Lauramine Oxide, Myristamidopropylamine Oxide, Myristamine Oxide, Palmitamidopropylamine Oxide, Palmitamine Oxide, PEG-3 Lauramine Oxide, Stearamidopropylamine Oxide, Stearamine Oxide, Tallowamidopropylamine Oxide, Tallowamine Oxide, Undecylenamidopropylamine Oxide.

Besonders bevorzugt als amphotere Tenside enthaltene Alkylamphoglycinate sind ausgewählt aus der Gruppe bestehend aus Caproamphoglycinate, Capryloamphoglycinate, Cocoamphoglycinate, Isostearoamphoglycinate, Lauroamphoglycinate, Myristoamphoglycinate, Oleoamphoglycinate, Stearoamphoglycinate, Tallowamphoglycinate, Undecylenoamphoglycinate.

Besonders bevorzugt als amphotere Tenside enthaltene Alkyliminodiacetate oder Alkyliminodipropionate sind ausgewählt aus der Gruppe bestehend aus Disodium Cocaminopropyl Iminodiacetate, Disodium Hydroxyethyliminodiacetate, Disodium Lauriminodiacetate, Disodium Lauriminodipropionate, Disodium Steariminodipropionate, Disodium Tallowiminodipropionate, Sodium C12-15 Alkoxypropyl Iminodipropionate, Sodium Cocoiminodiacetate, Sodium Lauriminodipropionate.

Besonders bevorzugt als amphotere Tenside enthaltene Alkylamphopropylsulfonate sind ausgewählt aus der Gruppe bestehend aus
Sodium Cocoamphohydroxypropylsulfonate, Sodium
Lauroamphohydroxypropylsulfonate, Sodium Oleoamphohydroxypropylsulfonate, Sodium Stearoamphohydroxypropylsulfonate, Disodium
Lauriminobishydroxypropylsulfonate, Disodium
Cocoamphocarboxyethylhydroxypropylsulfonate, Sodium
Caproamphohydroxypropylsulfonate, Sodium Capryloamphohydroxypropylsulfonate.

Ganz besonders bevorzugt als amphoteres Tensid enthalten ist Cocamidopropyl Betaine.

Bevorzugt in der erfindungsgemäßen Zusammensetzung enthaltene anionische Tenside sind ausgewählt aus der Gruppe umfassend, bevorzugt bestehend aus, Alkylsulfaten, Alkylethersulfaten, gegebenenfalls alkoxylierteSulfosuccinaten, gegebenenfalls alkoxylierteMethylsulfosuccinaten, gegebenenfalls alkoxylierte Sulfonaten, gegebenenfalls alkoxylierte Glycinaten, gegebenenfalls alkoxylierte Glutamaten, gegebenenfalls alkoxylierte Isethionaten, gegebenenfalls alkoxylierte Carboxylaten, gegebenenfalls alkoxylierte Anisate, gegebenenfalls alkoxylierte Levulinate, gegebenenfalls alkoxylierte Tartrate, gegebenenfalls alkoxylierte Lactylate, gegebenenfalls alkoxylierte Taurate, gegebenenfalls alkoxylierte Alaninate, gegebenenfalls alkoxylierte Phosphate, gegebenenfalls alkoxylierte Sulfoacetate, gegebenenfalls alkoxylierte Sulfosuccinamate, gegebenenfalls alkoxylierte Sarcosinaten und gegebenenfalls alkoxylierte Phosphonaten. In diesem Zusammenhang sind Dialkylsulfosuccinate von den Sulfosuccinaten und Dialkylmethylsulfosuccinate von den Methylsulfosuccinaten ausgenommen.

Bevorzugt in der erfindungsgemäßen Zusammensetzung als anionisches Tensid enthaltene Alkylsulfate oder Alkylethersulfate sind ausgewählt aus der Gruppe bestehend aus C4- bis C24-, bevorzugt C6- bis C18-, besonders bevorzugt C8- bis C14-, Alkylsulfaten und Alkylethersulfaten. Die Alkylreste können linear oder verzweigt sein, wobei lineare bevorzugt sind. Geeignete verzweigte Alkylreste umfassen Methyldecyl Gruppen, Methylundecyl-Gruppen, Methyldodecyl-Gruppen Ethyldecyl-Gruppen, Ethylundecyl-Gruppen und Ethyldodecyl-Gruppen, wie beispielsweise 1-Methyldecyl-, 1-Methylundecyl, 1-Methyldodecyl-, 1-Ethyldecyl, 1-Ethylundecyl und 1-Ethyldodecyl.
Der Zusatz einer Alkyl-oder Alkenylgruppe mit dem Suffix "eth" beschreibt allgemein die Zugabe von einem oder mehreren Ethylenoxid-Einheiten, beispielsweise bezieht sich Trideceth auf eine ethoxylierte Tridecylgruppe, und das Suffix "-n", wobei n eine ganze Zahl ist, die Anzahl von solchen Ethylenoxideinheiten pro Gruppe, beispielsweise "Trideceth-3" bezeichnet eine Gruppe ethoxylierter Tridecylalkohol mit 3 Ethylenoxid-Einheiten pro Tridecylgruppe.
In einer bevorzugten Ausführungsform ist das Alkylsulfat oder Alkylethersulfat ausgewählt aus,
Ammonium C12-15 Alkyl Sulfate, Ammonium C12-16 Alkyl Sulfate, Ammonium Capryleth Sulfate, Ammonium Cocomonoglyceride Sulfate, Ammonium Coco-Sulfate, Ammonium C12-15 Pareth Sulfate, Ammonium Laureth Sulfate, Ammonium Laureth-5 Sulfate, Ammonium Laureth-7 Sulfate, Ammonium Laureth-9 Sulfate, Ammonium Laureth-12 Sulfate, Ammonium Lauryl Sulfate, Ammonium Myreth Sulfate, Ammonium Myristyl Sulfate, Ammonium Nonoxynol-4 Sulfate, Ammonium Nonoxynol-30 Sulfate, Ammonium Palm Kernel Sulfate, Ammonium Trideceth Sulfate, DEA-C12-13 Alkyl Sulfate, DEA-C12-15 Alkyl Sulfate, DEA-Cetyl Sulfate, DEA-C12-13 Pareth-3 Sulfate, DEA-Laureth Sulfate, DEA-Lauryl Sulfate, DEA-Myreth Sulfate, DEA-Myristyl Sulfate, DEA-Trideceth Sulfate, Diethylamine Laureth Sulfate, Magnesium Coceth Sulfate, Magnesium Coco-Sulfate, Magnesium Laureth Sulfate, Magnesium Laureth-5 Sulfate, Magnesium Laureth-8 Sulfate, Magnesium Laureth-16 Sulfate, Magnesium Lauryl Sulfate, Magnesium Myreth Sulfate, Magnesium Oleth Sulfate, Magnesium PEG-3 Cocamide Sulfate, Magnesium/TEA-Coco-Sulfate, MEA-Laureth Sulfate, MEA-Lauryl Sulfate, MEA-Trideceth Sulfate, MIPA C12-15 Pareth Sulfate, MIPA-Laureth Sulfate, MIPA-Lauryl Sulfate, MIPA-Trideceth Sulfate, Mixed Isopropanolamines Lauryl Sulfate, Potassium Laureth Sulfate, Potassium Lauryl Sulfate, Sodium C8-10 Alkyl Sulfate, Sodium C10-16 Alkyl Sulfate, Sodium C11-15 Alkyl Sulfate, Sodium C12-13 Alkyl Sulfate, Sodium C12-15 Alkyl Sulfate, Sodium C12-18 Alkyl Sulfate, Sodium C16-20 Alkyl Sulfate, Sodium Caprylyl Sulfate, Sodium Cetearyl Sulfate, Sodium Cetyl Sulfate, Sodium Cholesteryl Sulfate, Sodium Coceth Sulfate, Sodium Coceth-30 Sulfate, Sodium Coco/Hydrogenated Tallow Sulfate, Sodium Cocomonoglyceride Sulfate, Sodium Coco-Sulfate, Sodium C9-15 Pareth-3 Sulfate, Sodium C10-15 Pareth Sulfate, Sodium C10-16 Pareth-2 Sulfate, Sodium C12-13 Pareth Sulfate, Sodium C12-14 Pareth-3 Sulfate, Sodium C12-15 Pareth Sulfate, Sodium C12-15 Pareth-3 Sulfate, Sodium C13-15 Pareth-3 Sulfate, Sodium C12-14 Sec-Pareth-3 Sulfate, Sodium Deceth Sulfate, Sodium Decyl Sulfate, Sodium Ethylhexyl Sulfate, Sodium Laneth Sulfate, Sodium Laureth Sulfate, Sodium Laureth-5 Sulfate, Sodium Laureth-7 Sulfate, Sodium Laureth-8 Sulfate, Sodium Laureth-12 Sulfate, Sodium Laureth-40 Sulfate, Sodium Lauryl Sulfate, Sodium/MEA-PEG-3 Cocamide Sulfate, Sodium Myreth Sulfate, Sodium Myristyl Sulfate, Sodium Nonoxynol-1 Sulfate, Sodium Nonoxynol-3 Sulfate, Sodium Nonoxynol-4 Sulfate, Sodium Nonoxynol-6 Sulfate, Sodium Nonoxynol-8 Sulfate, Sodium Nonoxynol-10 Sulfate, Sodium Nonoxynol-25 Sulfate, Sodium Octoxynol-2 Sulfate, Sodium Octoxynol-6 Sulfate, Sodium Octoxynol-9 Sulfate, Sodium Oleth Sulfate, Sodium Oleyl Sulfate, Sodium PEG-4 Cocamide Sulfate, Sodium PEG-4 Lauramide Sulfate, Sodium Stearyl Sulfate, Sodium Tallow Sulfate, Sodium/TEA C12-13 Pareth-3 Sulfate, Sodium Trideceth Sulfate, Sodium Tridecyl Sulfate, Sulfated Castor Oil, Sulfated Coconut Oil, Sulfated Glyceryl Oleate, Sulfated Olive Oil, Sulfated Peanut Oil, TEA-C10-15 Alkyl Sulfate, TEA-C11-15 Alkyl Sulfate, TEA-C12-13 Alkyl Sulfate, TEA-C12-14 Alkyl Sulfate, TEA-C12-15 Alkyl Sulfate, TEA-Coco-Sulfate, TEA-C11-15 Pareth Sulfate, TEA-C12-13 Pareth-3 Sulfate, TEA-Laneth-5 Sulfate, TEA-Laureth Sulfate, TEA-Lauryl Sulfate, TEA-Oleyl Sulfate, TEA-PEG-3 Cocamide Sulfate, TEA-Trideceth Sulfate, TIPA-Laureth Sulfate, TIPA-Lauryl Sulfate, wobei Sodium Laureth Sulfate ganz besonders bevorzugt ist.

Bevorzugt in der erfindungsgemäßen Zusammensetzung als anionisches Tensid enthaltene gegebenenfalls alkoxylierte Sulfosuccinate und/oder Methylsulfosuccinate sind ausgewählt aus der Gruppe bestehend aus gegebenenfalls alkoxylierten C4- bis C24-, bevorzugt C6- bis C18-, besonders bevorzugt C8- bis C14-, Sulfosuccinaten und/oder Methylsulfosuccinaten. Die Alkylreste können linear oder verzweigt sein, wobei lineare bevorzugt sind. Alkoxylierte Sulfosuccinate und/oder Methylsulfosuccinate können insbesondere einen Alkoxylierungsgrad zwischen 1 und 10, besonders bevorzugt zwischen 2 und 5 aufweisen.

Die Alkoxygruppe ist bevorzugt ausgewählt aus ethoxy.

Besonders bevorzugt enthaltene gegebenenfalls alkoxylierte Sulfosuccinate sind ausgewählt aus der Gruppe bestehend aus Disodium Laureth Sulfosuccinate, Disodium C12-14 Pareth-1 Sulfosuccinate, Disodium C12-14 Pareth-2 Sulfosuccinate, Disodium C12-14 Sec-pareth-12 Sulfosuccinate, Disodium C12-14 Sec-pareth-3 Sulfosuccinate, Disodium C12-14 Sec-pareth-5 Sulfosuccinate, Disodium C12-14 Sec-pareth-7 Sulfosuccinate, Disodium C12-14 Sec-pareth-9 Sulfosuccinate, Disodium C12-14 Pareth Sulfosuccinate, Di-Triethanolamine Oleamido PEG-2 Sulfosuccinate, Disodium Oleamido PEG-2 Sulfosuccinate, Disodium Cocamido Monoisopropanolamine PEG-4 Sulfosuccinate, Disodium Cocamido PEG-4 Sulfosuccinate, Disodium Coceth-3 Sulfosuccinate, Disodium Cocoyl Butyl Gluceth-10 Sulfosuccinate, Disodium Deceth-5 Sulfosuccinate, Disodium Deceth-6 Sulfosuccinate, Disodium Laneth-5 Sulfosuccinate, Disodium Lauramido PEG-2 Sulfosuccinate, Disodium Lauramido PEG-5 Sulfosuccinate, Disodium Laureth Sulfosuccinate, Disodium Laureth-12 Sulfosuccinate, Disodium Laureth-6 Sulfosuccinate, Disodium Laureth-9 Sulfosuccinate, Disodium Oleamido PEG-2 Sulfosuccinate, Disodium Oleth-3 Sulfosucciante, Disodium Palmitamido PEG-2 Sulfosuccinate, Disodium PEG-5 Laurylcitrate Sulfosuccinate, Disodium PEG-8 Palm Glycerides Sulfosuccinate, Disodium Sitostereth-14 Sulfosuccinate, Disodium Undecylenamide PEG-2 Sulfosuccinate, Magnesium Laureth-3 Sulfosuccinate, Monoethanolamine Laureth-2 sulfosuccinate, Diammonium C12-14 Pareth-1 Sulfosuccinate, Diammonium C12-14 Pareth-2 Sulfosuccinate, Diammonium C12-14 Sec-pareth-12 Sulfosuccinate, Diammonium C12-14 Sec-pareth-3 Sulfosuccinate, Diammonium C12-14 Sec-pareth-5 Sulfosuccinate, Diammonium C12-14 Sec-pareth-7 Sulfosuccinate, Diammonium C12-14 Sec-pareth-9 Sulfosuccinate, Diammonium C12-14 Pareth Sulfosuccinate, Di-Triethanolamine Oleamido PEG-2 Sulfosuccinate, Diammonium Oleamido PEG-2 Sulfosuccinate, Diammonium Cocamido Monoisopropanolamine PEG-4 Sulfoscuccinate, Diammonium Cocamido PEG-4 Sulfoscuccinate, Diammonium Coceth-3 Sulfosucciante, Diammonium Cocoyl Butyl Gluceth-10 Sulfosuccinate, Diammonium Deceth-5 Sulfosuccinate, Diammonium Deceth-6 Sulfosuccinate, Diammonium Laneth-5 Sulfosuccinate, Diammonium Lauramido PEG-2 Sulfosuccinate, Diammonium Lauramido PEG-5 Sulfosuccinate, Diammonium Laureth Sulfosuccinate, Diammonium Laureth-12 Sulfosuccinate, Diammonium Laureth-6 Sulfosuccinate, Diammonium Laureth-9 Sulfosuccinate, Diammonium Oleamido PEG-2 Sulfosuccinate, Diammonium Oleth-3 Sulfosucciante, Diammonium Palmitamido PEG-2 Sulfosuccinate, Diammonium PEG-5 Laurylcitrate Sulfosuccinate, Disodium PEG-8 Palm Glycerides Sulfosuccinate, Diammonium Sitostereth-14 Sulfosuccinate, Diammonium Undecylenamide PEG-2 Sulfosuccinate, Ammonium Lauryl Sulfosuccinate, Diammonium Lauramido-MEA Sulfosuccinate, Diammonium Lauryl Sulfosuccinate, Dipotassium Lauryl Sulfosuccinate, Disodium Babassuamido MEA-Sulfosuccinate, Disodium Cetearyl Sulfosuccinate, Disodium Cetyl Sulfosuccinate, Disodium Cocamido MEA-Sulfosuccinate, Disodium Cocamido MIPA-Sulfosuccinate, Disodium Coco-Glucoside Sulfosuccinate, Disodium Coco-Sulfosuccinate, Disodium Hydrogenated Cottonseed Glyceride Sulfosuccinate, Disodium Isodecyl Sulfosuccinate, Disodium Isostearamido MEA-Sulfosuccinate, Disodium Isostearamido MIPA-Sulfosuccinate, Disodium Isostearyl Sulfosuccinate, Disodium Lauramido MEA-Sulfosuccinate, Disodium Lauramido MIPA Glycol Sulfosuccinate, Disodium Lauryl Sulfosuccinate, Disodium Myristamido MEA-Sulfosuccinate, Disodium Oleamido MEA-Sulfosuccinate, Disodium Oleamido MIPA-Sulfosuccinate, Disodium Oleyl Sulfosuccinate, Disodium Polyglyceryl-3 Caprate/Caprylate Sulfosuccinate, Disodium Ricinoleamido MEA-Sulfosuccinate,
Disodium Stearamido MEA-Sulfosuccinate, Disodium Stearyl Sulfosuccinate, Disodium Tallamido MEA-Sulfosuccinate, Disodium Tallowamido MEA-Sulfosuccinate, Disodium Tridecylsulfosuccinate, Disodium Undecylenamido MEA-Sulfosuccinate, wobei Disodium Laureth Sulfosuccinate ganz besonders bevorzugt ist.

Bevorzugt in der erfindungsgemäßen Zusammensetzung als anionisches Tensid enthaltene gegebenenfalls alkoxylierte Sulfonate sind ausgewählt aus der Gruppe bestehend aus
Sodium C14-16 Olefin Sulfonate, Sodium C12-15 Pareth-15 Sulfonate, Sodium C14 - 17 Alkyl sec. Sulfonate, Sodium C14 Olefin Sulfonate, Ammonium Cumenesulfonate, Ammonium Dodecylbenzenesulfonate, Calcium Dodecylbenzenesulfonate, DEA-Dodecylbenzenesulfonate, DEA-Methyl Myristate Sulfonate, Disodium Decyl Phenyl Ether Disulfonate, Disodium Lauriminobishydroxypropylsulfonate, Disodium Lauryl Phenyl Ether Disulfonate, Isopropylamine Dodecylbenzenesulfonate, Magnesium Isododecylbenzenesulfonate, Magnesium Lauryl Hydroxypropyl Sulfonate, MEA-C10-13 Alkyl Benzenesulfonate, MIPA-Dodecylbenzenesulfonate, Potassium Dodecylbenzenesulfonate, Potassium Lauryl Hydroxypropyl Sulfonate, Sodium C13-17 Alkane Sulfonate, Sodium C14-18 Alkane Sulfonate, Sodium C10-13 Alkyl Benzenesulfonate, Sodium C9-22 Alkyl Sec Sulfonate, Sodium C14-17 Alkyl Sec Sulfonate, Sodium Caproylethylformyl Benzenesulfonate, Sodium Caprylyl PG-Sulfonate, Sodium Caprylyl Sulfonate, Sodium Cocoglucosides Hydroxypropylsulfonate, Sodium Cocoglyceryl Ether Sulfonate, Sodium Cocomonoglyceride Sulfonate, Sodium C12-14 Olefin Sulfonate, Sodium C14-16 Olefin Sulfonate, Sodium C14-18 Olefin Sulfonate, Sodium C16-18 Olefin Sulfonate, Sodium C14-15 Pareth-PG Sulfonate, Sodium C12-15 Pareth-3 Sulfonate, Sodium C12-15 Pareth-7 Sulfonate, Sodium C12-15 Pareth-15 Sulfonate, Sodium Decylbenzenesulfonate, Sodium Decylglucosides Hydroxypropylsulfonate, Sodium Dodecylbenzenesulfonate, Sodium Hydroxypropyl Palm Kernelate Sulfonate,
Sodium Lauroyl Hydroxypropyl Sulfonate, Sodium Laurylglucosides Hydroxypropylsulfonate, Sodium Methyl Laurate Sulfonate, Sodium Methyl Myristate Sulfonate, Sodium Methyl Palmitate Sulfonate, Sodium Methyl Stearate Sulfonate, Sodium Palm Glyceride Sulfonate, Sodium Phenylnonanoate Sulfonate, Sodium Tridecylbenzenesulfonate, TEA C14-17 Alkyl Sec Sulfonate, TEA-Dodecylbenzenesulfonate, TEA-Tridecylbenzenesulfonate.

Bevorzugt in der erfindungsgemäßen Zusammensetzung als anionisches Tensid enthaltene gegebenenfalls alkoxylierte Glycinate sind ausgewählt aus der Gruppe bestehend aus
Sodium Cocoyl Glycinate, Potassium Cocoyl Glycinate, Sodium Lauroyl Glycinate, Sodium Lauryl Diethylenediaminoglycinate, TEA-Cocoyl Glycinate.

Bevorzugt in der erfindungsgemäßen Zusammensetzung als anionisches Tensid enthaltene gegebenenfalls alkoxylierte Glutamate sind ausgewählt aus der Gruppe bestehend aus
Sodium Cocoyl Glutamate, Disodium Cocoyl Glutamate, Sodium Lauroyl Glutamate, Sodium Cocoyl Hydrolyzed Wheat Protein Glutamate, Dipotassium Capryloyl Glutamate, Dipotassium Undecylenoyl Glutamate, Disodium Capryloyl Glutamate, Disodium Cocoyl Glutamate, Disodium Hydrogenated Tallow Glutamate, Disodium Lauroyl Glutamate, Disodium Stearoyl Glutamate, Disodium Undecylenoyl Glutamate, Potassium Capryloyl Glutamate, Potassium Cocoyl Glutamate, Potassium Lauroyl Glutamate, Potassium Myristoyl Glutamate, Potassium Stearoyl Glutamate, Potassium Undecylenoyl Glutamate, Sodium Capryloyl Glutamate, Sodium Cocoyl Glutamate, Sodium Cocoyl/Hydrogenated Tallow Glutamate, Sodium Cocoyl Hydrolyzed Wheat Protein Glutamate, Sodium Cocoyl/Palmoyl/Sunfloweroyl Glutamate, Sodium Hydrogenated Tallowoyl Glutamate, Sodium Lauroyl Glutamate, Sodium Myristoyl Glutamate, Sodium Olivoyl Glutamate, Sodium Palmoyl Glutamate, Sodium Stearoyl Glutamate, Sodium Undecylenoyl Glutamate, TEA-Cocoyl Glutamate, TEA-Hydrogenated Tallowoyl Glutamate, TEA-Lauroyl Glutamate.

Bevorzugt in der erfindungsgemäßen Zusammensetzung als anionisches Tensid enthaltene gegebenenfalls alkoxylierte Isethionate sind ausgewählt aus der Gruppe bestehend aus
Sodium Lauroyl Methyl Isethionate, Sodium Cocoyl Isethionate, Ammonium Cocoyl Isethionate, Sodium Cocoyl Isethionate, Sodium Hydrogenated Cocoyl Methyl Isethionate, Sodium Lauroyl Isethionate, Sodium Lauroyl Methyl Isethionate, Sodium Myristoyl Isethionate, Sodium Oleoyl Isethionate, Sodium Oleyl Methyl Isethionate, Sodium Palm Kerneloyl Isethionate, Sodium Stearoyl Methyl Isethionate.

Bevorzugt in der erfindungsgemäßen Zusammensetzung als anionisches Tensid enthaltene gegebenenfalls alkoxylierte Carboxylate sind ausgewählt aus der Gruppe bestehend aus
Trideceth-7 Carboxylic Acid, Sodium Laureth-13 Carboxylate, Sodium Laureth-4 Carboxylate, Laureth-11 Carboxylic Acid, Laureth-5 Carboxylic Acid, Sodium Laureth-5 Carboxylate, Ammonium Laureth-6 Carboxylate, Ammonium Laureth-8 Carboxylate, Capryleth-4 Carboxylic Acid, Capryleth-6 Carboxylic Acid, Capryleth-9 Carboxylic Acid, Ceteareth-25 Carboxylic Acid, Cetyl C12-15 Pareth-8 Carboxylate, Cetyl C12-15-Pareth-9 Carboxylate, Cetyl PPG-2 Isodeceth-7 Carboxylate, Coceth-7 Carboxylic Acid, C9-11 Pareth-6 Carboxylic Acid, C9-11 Pareth-8 Carboxylic Acid, C11-15 Pareth-7 Carboxylic Acid, C12-13 Pareth-5 Carboxylic Acid, C12-13 Pareth-7 Carboxylic Acid, C12-13 Pareth-8 Carboxylic Acid, C12-13 Pareth-12 Carboxylic Acid, C12-15 Pareth-7 Carboxylic Acid, C12-15 Pareth-8 Carboxylic Acid, C12-15 Pareth-12 Carboxylic Acid, C14-15 Pareth-8 Carboxylic Acid, Deceth-7 Carboxylic Acid, Ethylhexeth-3 Carboxylic Acid, Hexeth-4 Carboxylic Acid, Isopropyl C12-15-Pareth-9 Carboxylate, Isopropyl PPG-2 Isodeceth-7 Carboxylate, Isosteareth-6 Carboxylic Acid, Isosteareth-11 Carboxylic Acid, Laureth-3 Carboxylic Acid, Laureth-4 Carboxylic Acid, Laureth-5 Carboxylic Acid, Laureth-6 Carboxylic Acid, Laureth-8 Carboxylic Acid, Laureth-10 Carboxylic Acid, Laureth-11 Carboxylic Acid, Laureth-12 Carboxylic Acid, Laureth-13 Carboxylic Acid, Laureth-14 Carboxylic Acid, Laureth-17 Carboxylic Acid, Magnesium Laureth-11 Carboxylate, MEA-Laureth-6 Carboxylate, MEA PPG-6 Laureth-7 Carboxylate, MEA-PPG-8-Steareth-7 Carboxylate, Myreth-3 Carboxylic Acid, Myreth-5 Carboxylic Acid, Oleth-3 Carboxylic Acid, Oleth-6 Carboxylic Acid, Oleth-10 Carboxylic Acid, PEG-2 Stearamide Carboxylic Acid, PEG-9 Stearamide Carboxylic Acid, Potassium Laureth-3 Carboxylate, Potassium Laureth-4 Carboxylate, Potassium Laureth-5 Carboxylate, Potassium Laureth-6 Carboxylate, Potassium Laureth-10 Carboxylate, Potassium Trideceth-3 Carboxylate, Potassium Trideceth-4 Carboxylate, Potassium Trideceth-7 Carboxylate, Potassium Trideceth-15 Carboxylate, Potassium Trideceth-19 Carboxylate, PPG-3-Deceth-2 Carboxylic Acid, Propyl C12-15 Pareth-8 Carboxylate, Sodium Capryleth-2 Carboxylate, Sodium Capryleth-9 Carboxylate, Sodium Ceteareth-13 Carboxylate, Sodium Ceteth-13 Carboxylate, Sodium C9-11 Pareth-6 Carboxylate, Sodium C11-15 Pareth-7 Carboxylate, Sodium C12-13 Pareth-5 Carboxylate, Sodium C12-13 Pareth-8 Carboxylate, Sodium C12-13 Pareth-12 Carboxylate, Sodium C12-15 Pareth-6 Carboxylate, Sodium C12-15 Pareth-7 Carboxylate, Sodium C12-15 Pareth-8 Carboxylate, Sodium C12-15 Pareth-12 Carboxylate, Sodium C14-15 Pareth-8 Carboxylate, Sodium C12-14 Sec-Pareth-8 Carboxylate, Sodium Deceth-2 Carboxylate, Sodium Hexeth-4 Carboxylate, Sodium Isosteareth-6 Carboxylate, Sodium Isosteareth-11 Carboxylate, Sodium Laureth-3 Carboxylate, Sodium Laureth-4 Carboxylate, Sodium Laureth-5 Carboxylate, Sodium Laureth-6 Carboxylate, Sodium Laureth-8 Carboxylate, Sodium Laureth-11 Carboxylate, Sodium Laureth-12 Carboxylate, Sodium Laureth-13 Carboxylate, Sodium Laureth-14 Carboxylate, Sodium Laureth-16 Carboxylate, Sodium Laureth-17 Carboxylate, Sodium Lauryl Glucose Carboxylate, Sodium Lauryl Glycol Carboxylate, Sodium PEG-6 Cocamide Carboxylate, Sodium PEG-8 Cocamide Carboxylate, Sodium PEG-3 Lauramide Carboxylate, Sodium PEG-4 Lauramide Carboxylate, Sodium PEG-7 Olive Oil Carboxylate, Sodium PEG-8 Palm Glycerides Carboxylate, Sodium Trideceth-3 Carboxylate, Sodium Trideceth-4 Carboxylate, Sodium Trideceth-6 Carboxylate, Sodium Trideceth-7 Carboxylate, Sodium Trideceth-8 Carboxylate, Sodium Trideceth-12 Carboxylate, Sodium Trideceth-15 Carboxylate, Sodium Trideceth-19 Carboxylate, Sodium Undeceth-5 Carboxylate, Trideceth-3 Carboxylic Acid, Trideceth-4 Carboxylic Acid, Trideceth-7 Carboxylic Acid, Trideceth-8 Carboxylic Acid, Trideceth-15 Carboxylic Acid, Trideceth-19 Carboxylic Acid und Undeceth-5 Carboxylic Acid.

Bevorzugt in der erfindungsgemäßen Zusammensetzung als anionisches Tensid enthaltene gegebenenfalls alkoxylierte Sarcosinate sind ausgewählt aus der Gruppe bestehend aus
Sodium Lauroyl Sarcosinate, Sodium Cocoyl Sarcosinate, Sodium Myristoyl Sarcosinate, TEA-Cocoyl Sarcosinate, Ammonium Cocoyl Sarcosinate, Ammonium Lauroyl Sarcosinate, Dimer Dilinoleyl Bis-Lauroylglutamate/Lauroylsarcosinate, Disodium Lauroamphodiacetate Lauroyl Sarcosinate, Isopropyl Lauroyl Sarcosinate, Potassium Cocoyl Sarcosinate, Potassium Lauroyl Sarcosinate, Sodium Cocoyl Sarcosinate, Sodium Lauroyl Sarcosinate, Sodium Myristoyl Sarcosinate, Sodium Oleoyl Sarcosinate, Sodium Palmitoyl Sarcosinate, TEA-Cocoyl Sarcosinate, TEA-Lauroyl Sarcosinate, TEA-Oleoyl Sarcosinate, TEA-Palm Kernel Sarcosinate.

Weitere Substanzen, die in der erfindungsgemäßen Zusammensetzung als anionisches Tensid enthalten sein können, sind ausgewählt aus der Gruppe bestehend aus Sodium Anisate, Sodium Levulinate, Sodium Coco-Glucoside Tartrate, Sodium Lauroyl Lactylate, Sodium Methyl Cocoyl Taurate, Sodium Methyl Lauroyl Taurate, Sodium Methyl Oleoyl Taurate, Sodium Cocoyl Alaninate, Sodium Laureth-4 Phosphate, Laureth-1 Phosphate, Laureth-3 Phosphate, Potassium Laureth-1 Phosphate, Sodium Lauryl Sulfoacetate und Sodium Coco Sulfoacetate, Disodium Stearyl Sulfosuccinamate, Disodium Tallow Sulfosuccinamate, Tetrasodium Dicarboxyethyl Stearyl Sulfosuccinamate, sowie deren alkoxylierte Varianten und Mischungen davon.

Es ist erfindungsgemäß bevorzugt, wenn die erfindungsgemäße Zusammensetzung zusätzlich eine Komponente D) 0 Gewichtsteile bis 15 Gewichtsteile, bevorzugt 5 Gewichtsteile bis 12 Gewichtsteile, besonders bevorzugt 7 Gewichtsteile bis 10 Gewichtsteile, mindestens eines hydrophoben Verdickers enthält.

Bevorzugte hydrophobe Verdicker, die in der erfindungsgemäßen Formulierung bevorzugt enthalten sind, sind ausgewählt aus der Gruppe enthaltend, bevorzugt bestehend aus,
Umsetzungsprodukte mindestens einer Fettsäure und/oder mindestens eines Fettsäureesters, wie zum Beispiel Fettsäureglycerinester, Fettsäuremethylester und Fettsäureethylester, mit mindestens einem primären oder sekundären Amin, wobei das Amin bevorzugt zusätzlich noch eine oder mehrere andere funktionale Gruppen wie beispielsweise Alkohole enthält, und
kationische Tenside, bei welchen bei einem pH-Wert von 7 und 20 °C mindestens 90 mol-% der Moleküle mindestens eine positiv geladene Gruppe aufweisen und bevorzugt keinen isolelektrischen Punkt von pH_{IEP}=2-12 bei 25°C aufweisen, wobei in einer wässrigen 10 millimolaren Kaliumchloridlösung als Hintergrundelektrolyt gemessen wird.

Bevorzugt in der erfindungsgemäßen Zusammensetzung enthaltene hydrophobe Verdicker sind ausgewählt aus der Gruppe umfassend, bevorzugt bestehend aus Alkamidmonoethanolaminen, Alkamiddieethanolaminen, Alkamidmonoisopropanolaminen, ethoxylierte Fettsäureamide, Fettalkoholethoxylate, Alkylquats und Alkylamidoalkylquats.

Bevorzugt in der erfindungsgemäßen Zusammensetzung als hydrophober Verdicker enthaltene Alkamidmonoethanolamine, Alkamiddieethanolamine und Alkamidmonoisopropanolamine sind ausgewählt aus der Gruppe enthaltend, bevorzugt bestehend aus, C4- bis C24-, bevorzugt C6- bis C18-, besonders bevorzugt C8- bis C14-, Alkamidmonoethanolaminen, Alkamiddieethanolaminen und Alkamidmonoisopropanolaminen. Die Alkylreste können linear oder verzweigt sein, wobei lineare bevorzugt sind. Besonders bevorzugt enthaltene Alkamidmonoethanolamine, Alkamiddieethanolamine und Alkamidmonoisopropanolamine sind ausgewählt aus der Gruppe bestehend aus Cocamide Monoethanolamine (=Cocamide MEA), Minkamide Diethanolamine, Oliveamide Monoethanolamine, Palm Kernelamide Monoethanolamine, Palm Kernelamide Diethanolamine, Palm Kernelamide Monoisopropanolamine, Palmamide Monoethanolamine, Palmamide Diethanolamine, Palmamide Monoisopropanolamine, Peanutamide Monoethanolamine, Peanutamide Diethanolamine, Peanutamide Monoisopropanolamine, Soyamide Diethanolamine, Tallamide Diethanolamine, Tallowamide Diethanolamine, Tallowamide Monoethanolamine, Cocamide Diethanolamine (=Cocamide DEA), Cocamide Monoispropanolamine (=Cocamide MIPA), Steramide Monoispropanolamine, Isostearamide Monoisopropanolamine, Lauramide Monoisopropanolamine, Linoleamide Monoisopropanolamine, Myristamide Monoisopropanolamine, Oleamide Monoisopropanolamine, Ricinoleamide Monoisopropanolamine, wobei Cocamide Monoethanolamine ganz besonders bevorzugt ist.

Erfindungsgemäß besonders bevorzugte Zusammensetzungen enthalten
A) 33 Gewichtsteile bis 47 Gewichtsteile Diethylhexyl SodiumSulfosuccinate,
B) 23 Gewichtsteile bis 28 Gewichtsteile Cocamidopropyl Betaine,
C) 23 Gewichtsteile bis 28 Gewichtsteile mindestens einer Substanz ausgewählt aus Sodium Laureth Sulfate und Disodium Laureth Sulfosuccinate, und
D) 7 Gewichtsteile bis 10 Gewichtsteile Cocamide MEA.

In einem Aspekt der Erfindung enthalten die erfindungsgemäßen Zusammensetzungen zusätzlich zu den Komponenten A) bis D) ein Lösungsmittel wie beispielsweise Wasser und/oder Propylenglykol, Dipropylenglycol, Ethanol, Isopropanol, Glycerin, insbesondere Wasser, bevorzugt in einer Menge von 20 Gew.-% bis 85 Gew.-%, bevorzugt von 30 Gew.-% bis 70 Gew.-%, besonders bevorzugt von 40 Gew.-% bis 50 Gew.-%, bezogen auf die Gesamtzusammensetzung.

Dies hat den technischen Effekt, dass die erfindungsgemäßen Zusammensetzungen sich einfacher ausformulieren lassen, eine bessere Verarbeitbarkeit aufweisen und lagerstabiler sind. In diese Aspekt der Erfindung ist es erfindungsgemäß bevorzugt, wenn die Summe der Komponenten A) bis D) in der erfindungsgemäßen Zusammensetzung in einer Menge von 5 Gew.-% bis 80 Gew.-%, bevorzugt von 8 Gew.-% bis 70 Gew.-%, besonders bevorzugt von 10 Gew.-% bis 60 Gew.-%, bezogen auf die Gesamtzusammensetzung enthalten ist.

Die erfindungsgemäßen Zusammensetzungen sind hervorragend zur Einarbeitung von großen Ölmengen unter Erhalt von strukturierten Formulierungen, insbesondere strukturierten kosmetischen Formulierungen, geeignet.
Somit ist ein weiterer Gegenstand der vorliegenden Erfindung eine strukturierte Formulierung, insbesondere eine kosmetische Formulierung, enthaltend
eine erfindungsgemäße Zusammensetzung und
E) mindestens ein kosmetisches Öl in einer Menge von 11 Gew.-% bis 74 Gew.-%, bevorzugt 16 Gew.-% bis 60 Gew.-%, besonders bevorzugt 21 Gew.-% bis 55 Gew.-%, wobei sich die Gewichtsprozente auf die Gesamtformulierung beziehen.

Unter dem Begriff "strukturierte Formulierung" im Zusammenhang mit der vorliegenden Erfindung ist eine Formulierung zu verstehen, welche über eine mit steigender Scherrate abfallende Viskosität im Scherratenbereich von 0,1 bis 100 s⁻¹ verfügt und welche eine Fließgrenze von >=1 mPa besitzt. Sowohl die Viskosität als auch die Fließgrenze wird durch Messung mit einem Rheometer, dessen Messachse in einem Luftlager gelagert ist, gemessen.

Die Viskosität fällt im angegeben Scherratenbereich um 1-10 Größenordnungen ab, wobei 2-6 Größenordnungen bevorzugt sind. Die Messungen wird mit einer Platte-Platte Geometrie mit einem Durchmesser von 40 mm bei 25°C durchgeführt.
Die Fließgrenze wird in Oszillation bei einer Frequenz von 1 Hz bei 25°C mit einer Platte-Platte Geometrie mit einem Durchmesser von 40 mm gemessen. Die Schubspannung wird dabei von 0,001 - 100 Pa variiert und als Kriterium für die Fließgrenze wird die Schubspannung genommen, bei der eine Abweichung von 5% des Speichermoduls vom Plateauwert des linear viskoelastischen Bereichs erreicht wird.

Bevorzugte erfindungsgemäße strukturierte Formulierungen weisen eine Fließgrenze von größer 0,001 Pa und besonders bevorzugt von 1 -100 Pa auf. Insbesondere bevorzugt ist in diesem Zusammenhang, dass die Fließgrenze von größer 0,001 Pa und bevorzugt von 1 -100 Pa in Abwesenheit von polymeren rheologischen Additiven in der erfindungsgemäßen Formulierung vorliegt.

Als kosmetische Öle können Substanzen wie Silikonöle, funktionalisierte Silikone, Mineralöle, Fettsäureester, natürliche Öle wie pflanzliche Öle, tierische Öle sowie deren Mischungen eingesetzt werden.

Bevorzugte erfindungsgemäße Formulierungen enthalten Cyclopentasiloxane, Cyclomethicone, Dimethicone, Dimethiconol, Amodimethicone, PEG/PPG Dimethicones, Cetyl Dimethicone, Stearyl Dimethicone, Stearoxy Dimethicone, Behenoxy Dimethicone, Polyisobutene, Petrolatum, Mineral Oil, Hydrogenated Polydodecene, Hydrogenated Polydecene, Polydecene, Isoamyl Cocoate, PPG-3 Myristyl Ether, PPG-11 Stearyl Ether, Dicaprylyl Ether, Dicaprylyl Carbonate, Cetearyl Isononanoate, Cetyl Ethylhexanoate, Diethyhexyl Carbonate, Cetyl Ricinoleate, Myristyl Myristate, Stearyl Heptanoate, Decyl Cocoate, Decyl Oleate, PPG-15 Stearyl Ether, Octyldodecanol, Isocetyl Palmitate, Cetearyl Ethylhexanoate, Ethylhexyl Palmitate, Ethylhexyl Stearate, Isopropyl Palmitate, PPG-14 Butyl Ether, Triisostearin, C12-15 Alkyl Benzoate, Phenoxyethyl Caprylate, Isopropyl Myristate, Caprylic/Capric Triglyceride, Sonnenblumenöl, Olivenöl, Arganöl, Mineralöl, Castoröl, Ricinusöl, Kakaoöl, Palmöl, Cocosöl, Avocadoöl, Mandelöl, Jojobaöl, Maisöl, Rapsöl, Sesamöl, Sojaöl, Weizenkeimöl, Walnußöl, Oleyl Erucate und deren Mischungen. Erfindungsgemäß bevorzugte Formulierungen enthalten die Komponenten A) bis E) in einem Gewichtsverhältnis der Summe der Komponenten A) bis D) zu Komponente E) von von 1 zu 1,1 bis 1 zu 10, bevorzugt von 1 zu 1,5 bis 1 zu 5, besonders bevorzugt von 1 zu 2 bis 1 zu 4.

Erfindungsgemäß entsprechend bevorzugte strukturierte Formulierungen enthalten oben als erfindungsgemäß bevorzugt genannte erfindungsgemäße Zusammensetzungen.

Bevorzugte erfindungsgemäße Formulierungen enthalten in Summe die Komponenten A) bis D) in einer Menge von 5 Gew.-% bis 35 Gew.-%, bevorzugt 8 Gew.-% bis 25 Gew.-%, besonders bevorzugt 11 Gew.-% bis 20 Gew.-%, wobei sich die Gewichtsprozente auf die Gesamtformulierung beziehen.

Bevorzugte erfindungsgemäße Formulierungen enthalten in Summe die Komponenten A) bis C) in einer Menge von 7 Gew.-% bis 16 Gew.-%, wobei sich die Gewichtsprozente auf die Gesamtformulierung beziehen.

Die erfindungsgemäßen Formulierungen sind bevorzugt wässrige Formulierungen, welche bevorzugt Wasser in einer Menge von 5 Gew.-% bis 80 Gew.-%, bevorzugt 20 Gew.-% bis 75 Gew.-%, besonders bevorzugt 30 Gew.-% bis 60 Gew.-%, enthalten, wobei sich die Gewichtsprozente auf die Gesamtformulierung beziehen.

Die erfindungsgemäßen Formulierungen sind insbesondere kosmetische Pflege- und Reinigungsformulierungen, insbesondere für Haut und Hautanhangsgebilde.

Unter dem Begriff "Pflegeformulierung" wird hier eine Formulierung verstanden, die den Zweck erfüllt, einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern.

Erfindungsgemäße Formulierungen umfassen somit Flüssigseifen, Duschgels, Ölbädern, Make-up Removern oder Shampoos, Schaumbäder, Haarshampoos, 2in1-Shampoos, Haarspülungen, Dauerwell- und Fixierlösungen, Haarfärbeshampoos, Haarfestiger, Haarkuren, Haarstyling-Zubereitungen, Fön-Lotionen, Schaumfestiger, Leave-In Conditioner, Haarglättungsmitteln, Glanzverbesserungsmitteln und Mittel zum Färben der Haare.

Erfindungsgemäße kosmetische Pflege- und Reinigungsformulierungen, können zum Beispiel mindestens eine zusätzliche Komponente enthalten, ausgewählt aus der Gruppe der
nicht-ionischen Tenside,
Emollients,
Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.
Substanzen, die als beispielhafte Vertreter der einzelnen Gruppen eingesetzt werden können, sind dem Fachmann bekannt und können beispielsweise der EP2273966A1 entnommen werden. Diese Patentanmeldung wird hiermit als Referenz eingeführt und gilt somit als Teil der Offenbarung.

Bezüglich weiterer fakultativer Komponenten sowie der eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Ein weiterer Gegenstand der vorliegenden Erfindung ist ein Verfahren zur Herstellung von strukturierten Formulierungen umfassend die Schritte
1) Bereitstellen einer erfindungsgemäßen Zusammensetzung
2) Mischen der erfindungsgemäßen Zusammensetzung mit E) mindestens einem kosmetischen Öl unter Erhalt einer strukturierten Formulierung enthaltend das Öl in einer Menge von 11 Gew.-% bis 74 Gew.-%, bevorzugt 16 Gew.-% bis 60 Gew.-%, besonders bevorzugt 21 Gew.-% bis 55 Gew.-%, wobei sich die Gewichtsprozente für das kosmetische Öl auf die Gesamtformulierung beziehen.

Erfindungsgemäß entsprechend bevorzugt wird das erfindungsgemäße Verfahren mit oben als erfindungsgemäß bevorzugt genannten erfindungsgemäßen Zusammensetzungen sowie mit oben als bevorzugt genannten Komponenten E) durchgeführt.

Erfindungsgemäß bevorzugte Verfahren werden mit erfindungsgemäßen Zusammensetzungen durchgeführt, die zusätzlich ein Lösungsmittel wie beispielsweise Wasser und/oder Propylenglykol, Dipropylenglycol, Ethanol, Isopropanol, Glycerin, insbesondere Wasser enthalten, bevorzugt in einer Menge von 20 Gew.-% bis 85 Gew.-%, bevorzugt von 30 Gew.-% bis 70 Gew.-%, besonders bevorzugt von 40 Gew.-% bis 50 Gew.-%, bezogen auf die in Verfahrensschritt 1) eingesetzte Gesamtzusammensetzung.

Erfindungsgemäß bevorzugt wird das erfindungsgemäße Verfahren derart durchgeführt, dass in der erhaltenen Formulierung Mengen der Komponenten A) bis D) von 5 Gew.-% bis 35 Gew.-%, bevorzugt 8 Gew.-% bis 25 Gew.-%, besonders bevorzugt 11 Gew.-% bis 20 Gew.-% enthalten sind, wobei sich die Gewichtsprozente auf die Gesamtformulierung beziehen.

Erfindungsgemäß bevorzugt wird das erfindungsgemäße Verfahren mit Mengen der Komponenten A) bis E) in einem Gewichtsverhältnis der Summe der Komponenten A) bis D) zu Komponente E) von von 1 zu 1,1 bis 1 zu 10, bevorzugt von 1 zu 1,5 bis 1 zu 5, besonders bevorzugt von 1 zu 2 bis 1 zu 4. durchgeführt.

Verfahrensschritt 2) des erfindungsgemäßen Verfahrens umfasst bevorzugt ein Emulgieren der enthaltenen Komponenten.

In einem bevorzugten erfindungsgemäßen Verfahren ist Wasser in einer Menge von 5 Gew.-% bis 80 Gew.-%, bevorzugt 20 Gew.-% bis 75 Gew.-%, besonders bevorzugt 30 Gew.-% bis 60 Gew.-%, wobei sich die Gewichtsprozente auf die durch das erfindungsgemäße Verfahren erhaltene Gesamtformulierung beziehen, enthalten.

Ein alternatives erfindungsgemäßes Verfahren zur Herstellung der erfindungsgemäßen strukturierten Formulierungen, welches einen weiteren Gegenstand der vorliegenden Erfindung darstellt, umfasst die Schritte
a) Bereitstellen einer Zusammensetzung enthaltend
   B) 15 Gewichtsteile bis 40 Gewichtsteile, bevorzugt 22 Gewichtsteile bis 32 Gewichtsteile, besonders bevorzugt 23 Gewichtsteile bis 28 Gewichtsteile, mindestens eines amphoteren Tensids,
   C) 15 Gewichtsteile bis 40 Gewichtsteile, bevorzugt 22 Gewichtsteile bis 32 Gewichtsteile, besonders bevorzugt 23 Gewichtsteile bis 28 Gewichtsteile, mindestens eines anionischen Tensids, und
   D) 0 Gewichtsteile bis 15 Gewichtsteile, bevorzugt 5 Gewichtsteile bis 12 Gewichtsteile, besonders bevorzugt 7 Gewichtsteile bis 10 Gewichtsteile, mindestens eines hydrophoben Verdickers,
b) Hinzufügen und Mischen der Komponenten A) und E) zu der Zusammensetzung aus Verfahrensschritt a)
   A) 20 Gewichtsteile bis 67 Gewichtsteile, bevorzugt 25 Gewichtsteile bis 57 Gewichtsteile, besonders bevorzugt 33 Gewichtsteile bis 47 Gewichtsteile, mindestens einer Substanz ausgewählt aus Dialkylsulfosuccinaten und Dialkylmethylsulfosuccinaten, und
   E) mindestens ein kosmetisches Öl in einer Menge von 11 Gew.-% bis 74 Gew.-%, bevorzugt 16 Gew.-% bis 60 Gew.-%, besonders bevorzugt 21 Gew.-% bis 55 Gew.-%, wobei sich die Gewichtsprozente für das kosmetische Öl auf die zu erhaltende Gesamtformulierung beziehen, und
   wobei die Summe der Gewichtsteile der Komponenten A) bis D) 100 Gewichtsteile ergeben.

Die Komponenten A) und E) können in Verfahrensschritt b) in beliebiger Reihenfolge erfindungsgemäß hinzugefügt werden. Es kann zuerst E), dann A) oder erst A), dann E) hinzugefügt werden. Die Komponenten A) und E) können auch mindestens teilweise gemischt werden und dann mindestens teilweise als Mischung in Verfahrensschritt b) erfindungsgemäß hinzugefügt werden.

Die in dem alternativen erfindungsgemäßen Verfahren eingesetzten Komponenten A) bis E) entsprechen den in den erfindungsgemäßen strukturierten Formulierungen enthaltenen Komponenten. Erfindungsgemäß entsprechend bevorzugt wird das erfindungsgemäße Verfahren mit oben als erfindungsgemäß bevorzugt genannten Komponenten A) bis D) der erfindungsgemäßen Zusammensetzungen sowie mit oben als bevorzugt genannten Komponenten E) der erfindungsgemäßen strukturierten Formulierungen durchgeführt.

Erfindungsgemäß bevorzugt wird das alternative erfindungsgemäße Verfahren derart durchgeführt, dass in der erhaltenen Formulierung Mengen der Komponenten A) bis D) von 5 Gew.-% bis 35 Gew.-%, bevorzugt 8 Gew.-% bis 25 Gew.-%, besonders bevorzugt 11 Gew.-% bis 20 Gew.-% enthalten sind, wobei sich die Gewichtsprozente auf die Gesamtformulierung beziehen.

Erfindungsgemäß bevorzugt wird das alternative erfindungsgemäße Verfahren mit Mengen der Komponenten A) bis E) in einem Gewichtsverhältnis der Summe der Komponenten A) bis D) zu Komponente E) von von 1 zu 1,1 bis 1 zu 10, bevorzugt von 1 zu 1,5 bis 1 zu 5, besonders bevorzugt von 1 zu 2 bis 1 zu 4durchgeführt.

Verfahrensschritt b) des alternativen erfindungsgemäßen Verfahrens umfasst bevorzugt ein Emulgieren der enthaltenen Komponenten.

In einem bevorzugten erfindungsgemäßen alternativen Verfahren ist Wasser in einer Menge von 5 Gew.-% bis 80 Gew.-%, bevorzugt 20 Gew.-% bis 75 Gew.-%, besonders bevorzugt 30 Gew.-% bis 60 Gew.-%, wobei sich die Gewichtsprozente auf die durch das erfindungsgemäße Verfahren erhaltene Gesamtformulierung beziehen, enthalten.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.
Folgende Abbildungen sind Bestandteil der Beispiele:

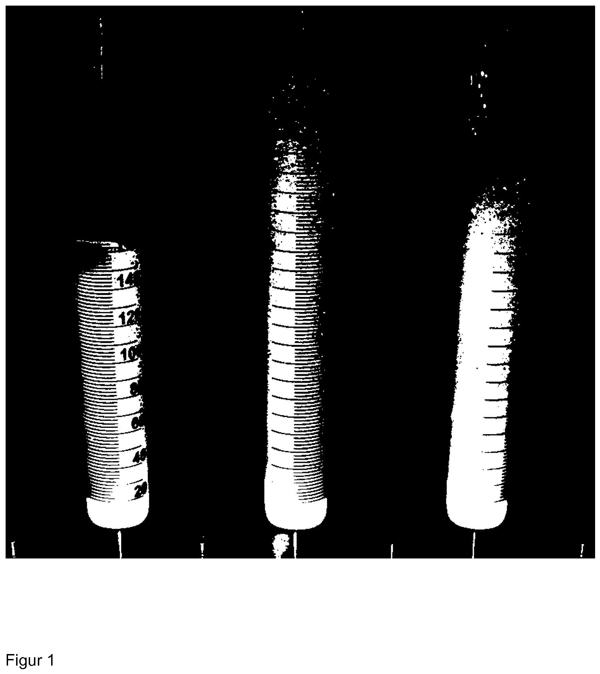

### Abbildung 1: Ergebnisse des Schaumtests im Standzylinder. Links (BW004):

### Formulierung enthaltend Tensidsystem nach Stand der Technik, mitte (235) und rechts (236): erfindungsgemäße Formulierungen

### Beispiele:

### Beispiele 1 bis 34, Hoch ölhaltige Formulierungen

| **Beispiel** | **Komponente A** | **Komponente C** | **Komponente B** | **Komponente D** | **Komponente E** | **Wasser** |
|---|---|---|---|---|---|---|
| **1** | 9 % Diethylhexyl Sodium Sulfosuccinate | 3 % Sodium Laureth Sulfate | 3% Cocamidopropyl Betaine | 0 | 11% Caprylic/Capric Triglyceride | ad 100 % |
| **2** | 9 % Diethylhexyl Sodium Sulfosuccinate | 3 % Disodium Laureth Sulfosuccinate | 3% Cocamidopropyl Betaine | 0 | 11 % Caprylic/Capric Triglyceride | ad 100 % |
| **3** | 9 % Diethylhexyl Sodium Sulfosuccinate | 3 % Disodium PEG-4 Cocamido MIPA-Sulfosuccinate | 3% Cocamidopropyl Betaine | 0 | 11 % Caprylic/Capric Triglyceride | ad 100 % |
| **4** | 9 % Diethylhexyl Sodium Sulfosuccinate | 3 % Sodium Laureth Sulfate | 3% Cocamidopropyl Betaine | 0 | 21 % Caprylic/Capric Triglyceride | ad 100 % |
| **5** | 9 % Diethylhexyl Sodium Sulfosuccinate | 3 % Sodium Laureth Sulfate | 3% Capryl/Caprami dopropyl Betaine | 0 | 21 % Caprylic/Capric Triglyceride | ad 100 % |
| **6** | 9 % Diethylhexyl Sodium Sulfosuccinate | 3 % Sodium Laureth Sulfate | 3 % Coco-Betaine | 0 | 21 % Caprylic/Capric Triglyceride | ad 100 % |
| **7** | 9 % Diethylhexyl Sodium Sulfosuccinate | 3 % Sodium Laureth Sulfate | 3 % Sodium Cocoamphoacet ate | 0 | 21 % Caprylic/Capric Triglyceride | ad 100 % |
| **8** | 9 % Diethylhexyl Sodium Sulfosuccinate | 3 % Disodium Laureth Sulfosuccinate | 3 % Sodium Cocoamphoacet ate | 0 | 21 % Caprylic/Capric Triglyceride | ad 100 % |
| **9** | 9 % Diethylhexyl Sodium Sulfosuccinate | 3 % Disodium PEG-5 Laureth Sulfosuccinate; Capryl/Caprami dopropyl Betaine | 3 % Sodium Cocoamphoacet ate | 0 | 10% Caprylic/Capric Triglyceride | ad 100 % |
| **10** | 9 % Diethylhexyl Sodium Sulfosuccinate | 3 % Sodium Laureth Sulfate | 3% Cocamidopropyl Betaine | 0 | 21 % C12-15 Alkyl Benzoate | ad 100 % |
| **11** | 9 % Diethylhexyl Sodium Sulfosuccinate | 3 % Sodium Laureth Sulfate | 3% Cocamidopropyl Betaine | 0 | 21 % Triisostearin | ad 100 % |
| **12** | 9 % Diethylhexyl Sodium Sulfosuccinate | 3 % Sodium Laureth Sulfate | 3% Cocamidopropyl Betaine | 0 | 21 % soybean oil | ad 100 % |
| **13** | 9 % Diethylhexyl Sodium Sulfosuccinate | 3 % Sodium Laureth Sulfate | 3% Cocamidopropyl Betaine | 0 | 21 % Helianthus Annuus Seed Oil | ad 100 % |
| **14** | 9 % Diethylhexyl Sodium Sulfosuccinate | 3 % Disodium Lauryl Sulfosuccinate | 3% Cocamidopropyl Betaine | 0 | 21 % Oleyl Erucate | ad 100 % |
| **15** | 9 % Diethylhexyl Sodium Sulfosuccinate | 3 % Sodium Lauryl Sulfate | 3% Cocamidopropyl Betaine | 0 | 21 % Oleyl Erucate | ad 100 % |
| **16** | 9 % Diethylhexyl Sodium Sulfosuccinate | 3 % Sodium Laureth Sulfate | 3% Cocamidopropyl Betaine | 1 % Cocamide MEA | 21 % Oleyl Erucate | ad 100 % |
| **17** | 9 % Diethylhexyl Sodium Sulfosuccinate | 3 % Sodium Laureth Sulfate | 3% Cocamidopropyl Betaine | 1 % Cocamide DEA | 21 % Oleyl Erucate | ad 100 % |
| **18** | 9 % Diethylhexyl Sodium Sulfosuccinate | 3 % Disodium Laureth Sulfosuccinate | 3% Cocamidopropyl Betaine | 1 % Cocamide MEA | 21 % Oleyl Erucate | ad 100 % |
| **19** | 8 % Diethylhexyl Sodium Sulfosuccinate | 3 % Sodium Laureth Sulfate | 3% Cocamidopropyl Betaine | 1 % Cocamide DEA | 40 % Oleyl Erucate | ad 100 % |
| **20** | 7 % Diethylhexyl Sodium Sulfosuccinate | 3 % Sodium Laureth Sulfate | 3% Cocamidopropyl Betaine | 1 % Cocamide MEA | 40 % Oleyl Erucate | ad 100 % |
| **21** | 5 % Diethylhexyl Sodium Sulfosuccinate | 3 % Sodium Laureth Sulfate | 3% Cocamidopropyl Betaine | 1 % Cocamide MEA | 50 % Oleyl Erucate | ad 100 % |
| **22** | 5 % Diethylhexyl Sodium Sulfosuccinate | 3 % Sodium Laureth Sulfate | 5% Cocamidopropyl Betaine | 1 % Cocamide MEA | 50 % Helianthus Annuus Seed Oil | ad 100 % |
| **23** | 5 % Diethylhexyl Sodium Sulfosuccinate | 3 % Sodium Laureth Sulfate | 3% Cocamidopropyl Betaine | 1 % Cocamide MEA | 50 % Helianthus Annuus Seed Oil | ad 100 % |
| **24** | 5 % Diethylhexyl Sodium Sulfosuccinate | 3 % Sodium Laureth Sulfate | 3% Cocamidopropyl Betaine | 1 % Cocamide MEA | 50 % soybean oil | ad 100 % |
| **25** | 5 % Diethylhexyl Sodium Sulfosuccinate | 3 % Sodium Laureth Sulfate | 3% Cocamidopropyl Betaine | 1 % Cocamide MEA | 50 % Helianthus Annuus Seed Oil | ad 100 % |
| **26** | 5 % Diethylhexyl Sodium Sulfosuccinate | 3 % Disodium Laureth Sulfosuccinate | 3% Cocamidopropyl Betaine | 1 % Cocamide MEA | 50 % Helianthus Annuus Seed Oil | ad 100 % |
| **27** | 3 % Diethylhexyl Sodium Sulfosuccinate | 3 % Sodium Laureth Sulfate | 3% Cocamidopropyl Betaine | 0 | 50 % Helianthus Annuus Seed Oil | ad 100 % |
| **28** | 4 % Diethylhexyl Sodium Sulfosuccinate | 3 % Sodium Laureth Sulfate | 3% Cocamidopropyl Betaine | 0 | 50 % Oleyl Erucate | ad 100 % |
| **29** | 4 % Diethylhexyl Sodium Sulfosuccinate | 3 % Sodium Laureth Sulfate | 3% Cocamidopropyl Betaine | 1 % Cocamide MEA | 50 % Oleyl Erucate | ad 100 % |

| **Beispiel** | **Komponente A** | **Komponente C** | **Komponente B** | **Komponente D** | **Komponente E** | **Additiv** | **Wasser** |
|---|---|---|---|---|---|---|---|
| **30** | 9 % Diethylhex yl Sodium Sulfosucci nate | 3 % Sodium Laureth Sulfate | 3 % Cocamidopr opyl Betaine | 0 | 21 % Caprylic/Cap ric Triglyceride | 0,2 % Polyquaternium-10 | ad 100 % |
| **31** | 8 % Diethylhex yl Sodium Sulfosucci nate | 3 % Sodium Laureth Sulfate | 3 % Cocamidopr opyl Betaine | 0 | 21 % Caprylic/Cap ric Triglyceride | 0,2% Polyquaternium-10 | ad 100 % |
| **32** | 9 % Diethylhex yl Sodium Sulfosucci nate | 3 % Sodium Laureth Sulfate | 3 % Cocamidopr opyl Betaine | 0 | 21 % Caprylic/Cap ric Triglyceride | 1 % Cetrimonium Chloride | ad 100 % |
| **33** | 3 % Diethylhex yl Sodium Sulfosucci nate | 3 % Sodium Laureth Sulfate | 3 % Cocamidopr opyl Betaine | 1 % Cocamide MEA | 50 % Helianthus Annuus Seed Oil | 0,2 % Polyquaternium-10 | ad 100 % |
| **34** | 3 % Diethylhex yl Sodium Sulfosucci nate | 3 % Disodium Laureth Sulfosuccina te | 3 % Cocamidopr opyl Betaine | 1 % Cocamide MEA | 50 % Helianthus Annuus Seed Oil | 0,2 % Polyquaternium-10 | ad 100 % |

### Beispiel 35, Technische Schaummessungen

### SITA R-2000 Foam Tester:

Zur Bewertung der Schaumeigenschaften und insbesondere der Anschäumbarkeit wurden Tests mit dem Sita Schaum Tester (SITA R-2000 Foam Tester, SITA Messtechnik GmbH, Germany) durchgeführt. Dazu wurden erfindungsgemäße Formulierungen im Vergleich zu einem Marktprodukt, nämlich dem Vergleichsbeispiel "DG Deeply Moisturizing Body Wash" (SdT) nach dem Stand der Technik geprüft. Das "DG Deeply Moisturizing Body Wash" beinhaltet eine strukturierte Zusammensetzung nach WO2008118381.

Es wurde bei T= 30 °C, mit Wasser der Härte 10 °dH (deutsche Härte), bei einem pH ∼6 und mit einer Umdrehungszahl von 1500 rpm gearbeitet.

Die Tabelle enthält die Schaumhöhen (in ml) nach verschiedenen Zeiten (10-100 sec) von erfindungsgemäßen Formulierungen, welche alle 3 % Sodium Laureth Sulfate, 3 % Cocamidopropyl Betaine, 1 % Cocamide MEA und 50 % Sonnenblumenöl und einen veränderlichen Anteil Diethylhexyl Sodium Sulfosuccinate enthalten.

| | **10 sec** | **20 sec** | **30 sec** | **40 sec** | **50 sec** | **60 sec** | **70 sec** | **80 sec** | **90 sec** | **100 sec** |
|---|---|---|---|---|---|---|---|---|---|---|
| **183** | 660,6 | 944,4 | 1019,6 | 1053 | 1069,6 | 1086,2 | 1108,4 | 1109,4 | 1108,4 | 1108,4 |
| **188** | 650,4 | 938,6 | 1023,8 | 1057,8 | 1074,2 | 1089,2 | 1115,6 | 1115,6 | 1115,2 | 1114,6 |
| **187** | 603 | 927 | 1017 | 1057 | 1078 | 1094 | 1112 | 1112 | 1111 | 1111 |
| **201** | 336,8 | 529,4 | 763,4 | 933,2 | 1018 | 1058,2 | 1078,8 | 1102,6 | 1102,2 | 1101,4 |
| **DG Body (SdT)** | 183,2 | 275,4 | 359,6 | 443,6 | 520,2 | 597,2 | 673 | 748,2 | 818,6 | 851 |

Die Ergebnisse zeigen zum einen, dass die Schaumhöhen der erfindungsgemäßen Zusammensetzungen denen der nach dem Stand der Technik weit überlegen sind. Darüberhinaus konnte gezeigt werden, dass durch eine größere Menge an Diethylhexyl Sodium Sulfosuccinate in den erfindungsgemäßen Formulierungen das Schaumvolumen vergrößert werden kann, Diethylhexyl Sodium Sulfosuccinate also in der Formulierung als gut schäumendes Tensid betrachtet werden kann.

| **Formulierungsbeispiele** | **183** | **188** | **187** | **201** |
|---|---|---|---|---|
| Texapon^{®} NSO-IS, BASF Cognis, 28%-ig, (INCI: Sodium Laureth Sulfate) | 10,7 % | 10,7 % | 10,7 % | 10,7 % |
| TEGO^{®} Sulfosuccinate DO 75, Evonik Industries AG, 75%-ig (INCI: Diethylhexyl Sodium Sulfosuccinate; Propylene Glycol) | 12,0 % | 9,3 % | 6,7 % | 4,0 % |
| TEGO^{®} Betain F 50, Evonik Industries AG, 38%-ig, (INCI: Cocamidopropyl Betaine) | 7,9 % | 7,9 % | 7,9 % | 7,9 % |
| REWOMID^{®} C 212, Evonik Industries AG, 100%-ig (INCI: Cocamide MEA) | 1,0 % | 1,0 % | 1,0 % | 1,0 % |
| Helianthus Annuus Seed Oil (Buttella Sonnenblumenöl, Brökelmann+ Co., Hamm, Deutschland) | 50,0 % | 50,0 % | 50,0 % | 50,0 % |
| Wasser, demineralisiert | 18,4 % | 21,1 % | 23,7 % | 26,4 % |

Da der genaue Anteil Öl in der SdT Formulierung nicht bekannt war, wurden auch Formulierungen mit unterschiedlichen Mengen an Öl (Oleyl Erucate, TEGOSOFT OER) im Sita Schaum Test verglichen. Die Formulierungen bestanden jeweils wieder aus 3 % Sodium Laureth Sulfate, 3 % Cocamidopropyl Betaine, 1 % Cocamide MEA und 7 % Diethylhexyl Sodium Sulfosuccinate.

| | **10 sec** | **20 sec** | **30 sec** | **40 sec** | **50 sec** | **60 sec** | **70 sec** | **80 sec** | **90 sec** | **100 sec** |
|---|---|---|---|---|---|---|---|---|---|---|
| **205** | 603 | 914,4 | 1007,2 | 1044 | 1067 | 1080,8 | 1088,8 | 1089 | 1089,6 | 1089,4 |
| **213** | 661,6 | 941,8 | 1023,4 | 1058,8 | 1073,2 | 1087 | 1112,2 | 1115,2 | 1114,4 | 1113,6 |
| **214** | 653 | 939 | 1024 | 1057 | 1076 | 1091 | 1116 | 1117 | 1117 | 1116 |
| **DG Body (SdT)** | 183,2 | 275,4 | 359,6 | 443,6 | 520,2 | 597,2 | 673 | 748,2 | 818,6 | 851 |

Es zeigte sich, dass die erfindungsgemäßen Formulierungen unabhängig vom Ölanteil nach kürzerer Zeit größere Schaumvolumina als die Formulierung nach SdT zeigen.

| **Formulierungsbeispiele** | **205** | **213** | **214** |
|---|---|---|---|
| Texapon^{®} NSO-IS, BASF Cognis, 28%-ig, (INCI: Sodium Laureth Sulfate) | 107 % | 10,7 % | 10,7 % |
| TEGO^{®} Sulfosuccinate DO 75, Evonik Industries AG, 75%-ig (INCI: Diethylhexyl Sodium Sulfosuccinate; Propylene Glycol) | 9,3 % | 9,3 % | 9,3 % |
| TEGO^{®} Betain F 50, Evonik Industries AG, 38%-ig, (INCI: Cocamidopropyl Betaine) | 7,9 % | 7,9 % | 7,9 % |
| REWOMID^{®} C 212, Evonik Industries AG, 100%-ig (INCI: Cocamide MEA) | 1,0 % | 1,0 % | 1,0 % |
| TEGOSOFT^{®} OER, Evonik Industries AG, 100%ig (INCI: Oleyl Erucate) | 50,0 % | 40,0 % | 30,0 % |
| Wasser, demineralisiert | 21,1 % | 31,1 % | 41,1 % |

### Schaumtest in Standzylindern

Drei verschraubbare und verbundene 250 mL Standzylinder wurden mit 10 Gramm Formulierung und 20 g Wasser befüllt. Durch Verrühren mit einem Glasstab wurde die Durchmischung der Formulierungen und dem Wasser gewährleistet. Nachdem die Standzylinder verschlossen wurden, wurde der Aufbau solange händisch geschüttelt, bis in einem der Standzylinder ein Volumen von 250 mL (Schaum und Flüssigkeit) erreicht war. Die Temperatur bei diesem Versuch betrug dabei 23°C. Die Apparatur wurde daraufhin auf eine waagerechte Fläche gestellt; nach 30 Sekunden wurde ein Bild aufgenommen, um die Schaumvolumina zu dokumentieren. Bei den Formulierungen handelte es sich sowohl um Formulierungen, die mit einer Tensidmischung gemäß dem Stand der Technik hergestellt wurden als auch um erfindungsgemäße Formulierungen. Die folgende Tabelle und Abbildung 1 fassen die Ergebnisse des Schaumtests im Zylinder zusammen.

| **Formulierungsbeispiele** | **BW004** | **235** | **236** |
|---|---|---|---|
| Texapon^{®} NSO-IS, BASF Cognis, 28%-ig, (INCI: Sodium Laureth Sulfate) | - | 10,7 % | 14,3 % |
| TEGO^{®} Sulfosuccinate DO 75, Evonik Industries AG, 75%-ig (INCI: Diethylhexyl Sodium Sulfosuccinate; Propylene Glycol) | - | 12,0 % | 9,3 % |
| TEGO^{®} Betain F 50, Evonik Industries AG, 38%-ig, (INCI: Cocamidopropyl Betaine) | - | 7,9 % | 10,5 % |
| REWOMID^{®} C 212, Evonik Industries AG, 100%-ig (INCI: Cocamide MEA) | - | 1,0 % | 1,0 % |
| Miracare 365/N, Rhodia, 40%ig (INCI: Water, Sodium Trideceth Sulfate, Sodium Lauroamphoacetate, Cocamide MEA, Sodium Chloride, Methylisothiazolinone | 35,0 % | - | - |
| Helianthus Annuus Seed Oil (Buttella Sonnenblumenöl, Brökelmann+ Co., Hamm, Deutschland) | 27,0 % | 27,0 % | 27,0 % |
| Hydroxypropyl Guar Hydroxypropyltrimonium Chloride (Jaguar C-162, Rhodia, 100%) | 0,1 % | 0,1 % | 0,1 % |
| Natriumchlorid-Lösung, 20 Gew% in Wasser (Natriumchlorid, Merck, 99,9 %) | 12,5 % | - | - |
| Wasser, demineralisiert | 25,4 % | 41,3 % | 37,8 % |
| Schaumvolumen | 160 mL | 250 mL | 200 mL |

Die Ergebnisse des Schaumtests im Standzylinder in Abbildung 1 zeigen, dass die erfindungsgemäßen Formulierungen besser schäumen als Formulierungen nach Stand der Technik. Außerdem konnte überraschenderweise gezeigt werden, dass erfindungsgemäße Formulierungen mit einem höheren Anteil an Diethylhexyl Sodium Sulfosuccinate als gut schäumende Tensidmischungen betrachtet werden können.

### Beispiel 36: Verbesserte sensorische Schaumeigenschatten der erfindungsgemäßen Formulierungen im Vergleich zum Stand der Technik

Zur Bewertung der Schaumeigenschaften und der Hautpflegeleistung der erfindungsgemäßen Formulierungen wurde ein sensorischer Handwaschtest im Vergleich zu einem Marktprodukt, nämlich dem Vergleichsbeispiel "DG Deeply Moisturizing Body Wash" (SdT) nach dem Stand der Technik durchgeführt. Das "DG Deeply Moisturizing Body Wash" beinhaltet eine strukturierte Zusammensetzung nach WO2008118381.

Eine Gruppe bestehend aus 10 trainierten Prüfpersonen wusch sich dabei definiert die Hände und bewertete Schaumeigenschaften und Hautgefühl anhand einer Notenskala von 1 (schlecht) bis 5 (sehr gut).

Die Zusammensetzungen der erfindungsgemäßen Formulierungen sind in Tabelle 01 aufgeführt:

**Tab. 01: Testformulierungen für den Handwaschtest:**

| **Formulierungsbeispiele** | **149** | **153** | **160** |
|---|---|---|---|
| Texapon^{®} NSO-IS, BASF Cognis, 28%-ig, (INCI: Sodium Laureth Sulfate) | 10,7 % | | |
| REWOPOL^{®} SB FA 30 B, Evonik Industries AG, 40%-ig (INCI: Disodium Laureth Sulfosuccinate) | | 7,5 % | |
| REWOPOL^{®} SB C 55, Evonik Industries AG, 40%-ig (INCI: Disodium PEG-5 Laurylcitrate Sulfosuccinate; Capryl/Capramidopropyl Betaine) | | | 7,5 % |
| TEGO^{®} Sulfosuccinate DO 75 Diethylhexyl Sodium Sulfosuccinate, Evonik Industries AG, 75%-ig (INCI: Diethylhexyl Sodium Sulfosuccinate; Propylene Glycol) | 12,0 % | 12,0 % | 12,0 % |
| TEGO^{®} Betain F 50, Evonik Industries AG, 38%-ig, (INCI: Cocamidopropyl Betaine) | 7,9 % | 7,9 % | 7,9 % |
| REWOMID^{®} C 212, Evonik Industries AG, 100%-ig (INCI: Cocamide MEA) | 1,0 % | 1,0 % | 1,0 % |
| TEGOSOFT^{®} OER, Evonik Industries AG, 100%ig (INCI: Oleyl Erucate) | 21,0 % | 21,0 % | 21,0 % |
| Wasser, demineralisiert | 47,4 % | 50,6 % | 50,6 % |

Die sensorischen Testergebnisse sind in Tabelle 02 zusammengefasst.

**Tab. 02: Ergebnisse des Handwaschtests:**

| **Testformulierung** | **149** | **153** | **160** | **SdT** |
|---|---|---|---|---|
| Anschäumverhalten | 3,1 | 3,4 | 2,3 | 1,3 |
| Schaumvolumen | 2,9 | 2,5 | 2,1 | 1,0 |
| Hautgefühl während des Waschens | 3,1 | 3,2 | 3,1 | 4,1 |
| Schaumcremigkeit | 2,1 | 2,2 | 2,3 | 1,1 |
| Abwaschbarkeit | 3,4 | 3,4 | 3,5 | 2,3 |
| Hautglätte | 1,9 | 1,8 | 2,3 | 2,6 |
| Hautweichheit | 2,6 | 2,6 | 2,8 | 3,1 |
| Hautglätte nach 3 min. | 3,8 | 3,3 | 3,6 | 3,8 |
| Hautweichheit nach 3 min. | 3,6 | 3,5 | 3,8 | 3,8 |

Anhand der Testergebnisse in Tabelle 02 wird ersichtlich, dass die erfindungsgemäßen Formulierungen in vielen Applikationseigenschaften im Vergleich zur Vergleichsformulierung SdT nach dem Stand der Technik überlegen sind. Vor diesem Hintergrund sind die Ergebnisse der erfindungsgemäßen Formulierungen als sehr gut zu bezeichnen und zeigen eine deutliche Verbesserung gegenüber dem Stand der Technik.
Anhand der Messwerte ist ersichtlich, dass die erfindungsgemäßen Formulierungen vor allem zu einer signifikanten Verbesserung, speziell bei Anschäumverhalten, Schaumvolumen, Schaumcremigkeit und der Abwaschbarkeit, führte.

**Weitere Formulierungsbeispiele:**

| | **37a** | **37b** | **37c** | **37d** | **37e** | **37f** | **37g** |
|---|---|---|---|---|---|---|---|
| **Wasser** | ad 100% | | | | | | |
| **Sodium Laureth Sulfate** | 3,0% | 3,0% | 3,0% | 3,0% | 3,0% | 3,0% | 3,0% |
| **Cocamidopropyl Betaine** | 3,0% | 3,0% | 3,0% | 3,0% | 3,0% | 3,0% | 3,0% |
| **Cocamide MEA** | 1,0% | 1,0% | 1,0% | 1,0% | 1,0% | 0,0% | 0,0% |
| **Diethylhexyl Sodium Sulfosuccinate** | 9,0% | 9,0% | 9,0% | 8,0% | 7,0% | 9,0% | 9,0% |
| **Capryl**/**Capric TriglycerideCaprylic**/**Capric Triglyceride** | 10,0% | 20,0% | 30,0% | 40,0% | 50,0% | 20,0% | 50,0% |

| | **38a** | **38b** | **38c** | **38d** | **38e** | **38f** | **38g** |
|---|---|---|---|---|---|---|---|
| **Wasser** | ad 100% | | | | | | |
| **Sodium Laureth Sulfate** | 3,0% | 3,0% | 3,0% | 3,0% | 5,0% | 3,0% | 3,0% |
| **Cocamidopropyl Betaine** | 3,0% | 3,0% | 3,0% | 3,0% | 3,0% | 3,0% | 3,0% |
| **Cocamide MEA** | 1,0% | 1,0% | 1,0 % | 1,0% | 1,0% | 0,0% | 0,0% |
| **Diethylhexyl Sodium Sulfosuccinate** | 4,0% | 5,0% | 7,0% | 9,0% | 5,0% | 5,0% | 7,0% |
| **Oleyl Erucate** | 50,0% | 50,0% | 50,0% | 50,0% | 50,0% | 50,0% | 50,0% |

| | **39a** | **39b** | **39c** | **39d** | **39e** | **39f** | **39g** |
|---|---|---|---|---|---|---|---|
| **Wasser** | ad 100% | | | | | | |
| **Sodium Laureth Sulfate** | 3,0% | 3,0% | 3,0% | 3,0% | 3,0% | 3,0% | 3,0% |
| **Cocamidopropyl Betaine** | 3,0% | 3,0% | 3,0% | 3,0% | 3,0% | 3,0% | 3,0% |
| **Cocamide MEA** | 0,0% | 0,0% | 0,0% | 0,0% | 0,0% | 1,0% | 1,0% |
| **Diethylhexyl Sodium Sulfosuccinate** | 5,0% | 5,0% | 5,0% | 5,0% | 5,0% | 5,0% | 5,0% |
| **Oleyl Erucate** | 40,0% | 40,0% | 40,0% | 40,0% | 40,0% | 40,0% | 40,0% |
| **Sodium Cocoyl Glutamate** | 1,0% | 1,0% | 1,0% | 0,0% | 0,0% | 0,0% | 0,0% |
| **Cocamide DEA** | 0,0% | 0,5% | 1,0% | 0,0% | 0,0% | 0,0% | 0,0% |

| | **40a** | **40b** | **40c** | **40d** | **40e** | **40f** | **40g** |
|---|---|---|---|---|---|---|---|
| **Wasser** | ad 100% | | | | | | |
| **Sodium Laureth Sulfate** | 3,0% | 3,0% | 3,0% | 3,0% | 3,0% | 3,0% | 3,0% |
| **Cocamidopropyl Betaine** | 3,0% | 3,0% | 3,0% | 3,0% | 3,0% | 3,0% | 3,0% |
| **Cocamide MEA** | 1,0% | 1,0% | 1,0% | 1,0% | 1,0% | 1,0% | 1,0% |
| **Diethylhexyl Sodium Sulfosuccinate** | 5,0% | 5,0% | 5,0% | 5,0% | 5,0% | 5,0% | 5,0% |
| **Oleyl Erucate** | 40,0% | 30,0% | 40,0% | 40,0% | 40,0% | 40,0% | 40,0% |
| **Glycerin** | 0,0% | 0,0% | 5,0% | 0,0% | 0,0% | 0,0% | 0,0% |
| **Polyquaternium-10** | 0,0% | 0,0% | 0,0% | 0,5% | 0,0% | 0,0% | 0,0% |
| **Hydroxypropyl Guar Hydroxypropyltrimonium Chloride** | 0,0% | 0,0% | 0,0% | 0,0% | 0,5% | 0,0% | 0,5% |
| **Silicone Quaternium-22** | 0,0% | 0,0% | 0,0% | 0,0% | 0,0% | 0,5% | 0,0% |
| **Dimethicone** | 0,0% | 0,0% | 0,0% | 0,5% | 0,0% | 0,0% | 0,0% |
| **Amodimethicone** | 0,0% | 0,0% | 0,0% | 0,0% | 0,0% | 0,0% | 0,5% |
| **Argania Spinosa Oil** | 0,0% | 10,0% | 0,0% | 0,0% | 0,0% | 0,0% | 0,0% |

| | **41a** | **41b** | **41c** | **41d** | **41e** | **41f** | **41g** |
|---|---|---|---|---|---|---|---|
| **Wasser** | ad 100% | | | | | | |
| **Sodium Laureth Sulfate** | 3,0% | 3,0% | 3,0% | 3,0% | 3,0% | 3,0% | 3,0% |
| **Cocamidopropyl Betaine** | 3,0% | 3,0% | 3,0% | 3,0% | 3,0% | 3,0% | 3,0% |
| **Cocamide MEA** | 0,0% | 1,0% | 1,0% | 1,0% | 1,0% | 1,0% | 1,0% |
| **Diethylhexyl Sodium Sulfosuccinate** | 5,0% | 5,0% | 5,0% | 5,0% | 5,0% | 5,0% | 5,0% |
| **Prunus Amygdalus Dulcis Oil** | 20,0% | 0,0% | 10,0% | 0,0% | 0,0% | 10,0% | 0,0% |
| **Olea Europaea Fruit Oil** | 5,0% | 5,0% | 0,0% | 0,0% | 20,0% | 10,0% | 0,0% |
| **Butyrospermum Parkii Butter Extract** | 5,0% | 20,0% | 5,0% | 0,0% | 10,0% | 0,0% | 0,0% |
| **Persea Gratissima Oil** | 0,0% | 0,0% | 10,0% | 30,0% | 0,0% | 20,0% | 0,0% |
| **Isostearamide MIPA** | 1,0% | 0,0% | 0,0% | 0,0% | 0,0% | 0,0% | 0,0% |
| **Zinc Pyrithione** | 0,0% | 0,1% | 0,0% | 0,0% | 0,0% | 0,0% | 0,0% |
| **Benzophenone-4** | 0,0% | 0,1% | 0,1% | 0,1% | 0,1% | 0,0% | 0,1% |
| **Tetrasodium EDTA** | 0,1% | 0,1% | 0,0% | 0,1% | 0,1% | 0,0% | 0,0% |
| **Caffeine** | 0,0% | 0,1% | 0,1% | 0,0% | 0,0% | 0,0% | 0,0% |
| **Hydrolyzed Keratin** | 0,0% | 0,0% | 0,1% | 0,0% | 0,0% | 0,1% | 0,2% |
| **Panthenol** | 0,1% | 0,1% | 0,1% | 0,1% | 0,1% | 0,1% | 0,0% |

| | **42a** | **42b** | **42c** | **42d** | **42e** | **42f** | **42g** |
|---|---|---|---|---|---|---|---|
| **Wasser** | ad 100% | | | | | | |
| **Sodium Laureth Sulfate** | 0,0% | 0,0% | 3,0% | 3,0% | 0,0% | 0,0% | 0,0% |
| **Cocamidopropyl Betaine** | 3,0% | 3,0% | 0,0% | 0,0% | 0,0% | 3,0% | 3,0% |
| **Cocamide MEA** | 0,0% | 1,0% | 1,0% | 1,0% | 1,0% | 1,0% | 1,0% |
| **Diethylhexyl Sodium Sulfosuccinate** | 5,0% | 5,0% | 5,0% | 5,0% | 5,0% | 5,0% | 5,0% |
| **Sodium Lauryl Sulfate** | 3,0% | 0,0% | 0,0% | 0,0% | 0,0% | 3,0% | 3,0% |
| **Ammonium Laureth Sulfate** | 0,0% | 3,0% | 3,0% | 0,0% | 0,0% | 0,0% | 0,0% |
| **Capryl/Capramidopropyl Betain** | 0,0% | 0,0% | 3,0% | 3,0% | 3,0% | 0,0% | 3,0% |
| **Disodium Lauryl Sulfosuccinate** | 0,0% | 0,0% | 0,0% | 0,0% | 3,0% | 3,0% | 0,0% |
| **Disodium PEG-4 Cocamido MIPA-Sulfosuccinate** | 0,0% | 0,0% | 0,0% | 0,0% | 0,0% | 0,0% | 3,0% |

| | **43a** | **43b** | **43c** | **43d** | **43e** | **43f** | **43g** | **43h** | **43i** | **43j** |
|---|---|---|---|---|---|---|---|---|---|---|
| **Wasser** | ad 100% | | | | | | | | | |
| **Diethylhexyl Sodium Sulfosuccinate** | 10,0 % | 7,5% | 6,5% | 6,0% | 9,0% | 7,0% | 9,0% | 6,0% | 8,5% | 6,5% |
| **Sodium Laureth Sulfate** | 3,5% | 3,0% | 2,5% | - | - | 2,5% | 1,5% | 2,6% | - | - |
| **Sodium Lauryl Sulfate** | - | - | 0,5% | 3,0% | 3,2% | - | 1,0% | 0,5% | 3,5% | 2,5% |
| **Cocamidopropyl Betaine** | - | 3,0% | 3,0% | 2,8% | 4,0% | 3,5% | - | 1,0% | 2,0% | 3,0% |
| **Sodium Cocoamphoacetate** | 3,0% | - | - | 0,5% | - | - | 3,0% | 2,0% | 3,5% | - |
| **Lauryl Glucoside** | - | - | - | - | 0,2% | - | - | - | - | - |
| **Coco-Glucoside** | - | 0,5% | - | - | 0,3% | - | 0,5% | - | - | - |
| **Sodium/Disodium Cocoyl Glutamate** | - | - | - | - | 0,2% | 1,0% | 1,0% | - | 0,3% | - |
| **Stearic Acid** | - | - | 0,1% | - | - | - | - | - | - | 0,2% |
| **Glycerin** | 0,5% | 1,0% | 0,5% | - | 0,3% | 0,4% | 1,0% | 0,4% | 0,4% | 0,2% |
| **Polyglyceryl-4 Caprate** | - | - | - | 1,0% | - | 0,5% | - | - | - | 0,5% |
| **Polyquaternium-10** | - | 0,2% | - | 0,1% | - | - | - | 0,2% | 0,2% | - |
| **Hydroxypropyl Guar Hydroxypropyltrimonium Chloride** | 0,2% | - | 0,3% | 0,2% | 0,2% | - | 0,2% | 0,1% | - | - |
| **Palmitamidopropyltrimoniu m Chloride** | - | 0,1% | 0,2% | - | - | - | - | - | - | 0,1% |
| **Silicone Quaternium-22** | - | - | 0,3% | - | 0,3% | - | - | - | - | - |
| **Dimethicone** | - | - | - | - | - | - | - | - | 0,1% | - |
| **Amodimethicone** | - | - | - | 0,5% | - | - | - | - | 0,5% | - |
| **Argania Spinosa Oil** | - | - | 0,2% | 0,1% | 0,1% | - | 0,2% | - | - | - |
| **Glycine Soja Oil** | 22% | - | - | - | 20% | 25% | 25% | 25% | 25% | 25% |
| **Simmondsia Chinensis (Jojoba) Seed Oil** | - | 35% | - | - | - | - | - | 5% | - | - |
| **Prunus Amygdalus Dulcis Oil** | - | - | 40% | - | - | - | - | 5% | 0,5% | - |
| **Olea Europaea Fruit Oil** | - | - | - | 45% | - | - | - | 5% | 0,5% | 20% |
| **Butyrospermum Parkii Butter Extract** | - | - | - | - | 15% | - | - | - | - | - |
| **Persea Gratissima Oil** | - | - | - | - | - | 15% | 2% | - | - | - |
| **Hydrogenated Castor Oil** | - | - | - | - | - | - | 8% | - | 0,5% | 0,5% |
| **Glycol Distearate** | - | 0,5% | - | - | 0,5% | - | 0,3% | - | - | - |
| **Isostearamide MIPA** | 1,0% | - | - | 1,5% | - | - | 0,2% | - | 1,0% | 0,5% |
| **Cocamide DEA** | - | - | 0,5% | - | - | 1,0% | - | - | - | - |
| **Sodium Chloride** | 0,3% | - | 0,3% | 0,5% | - | - | - | 0,2% | - | 0,3% |
| **PEG-200 Hydrogenated Glyceryl Palmate; PEG-7 Glyceryl Cocoate** | 0,2% | - | - | - | - | - | - | - | 0,5% | - |
| **Xanthan Gum** | - | - | - | - | - | 0,7% | - | - | - | - |
| **Cellulose** | - | - | - | 0,1% | - | 0,1% | - | 0,2% | 0,1% | - |
| **Zinc Pyrithione** | - | 0,1% | - | - | - | - | - | - | 0,1% | - |
| **Benzophenone-4** | - | 0,1% | 0,1% | 0,1% | 0,1% | - | 0,1% | - | 0,1% | - |
| **Tetrasodium EDTA** | 0,1% | 0,1% | - | 0,1% | 0,1% | - | - | - | 0,1% | - |
| **Hydrolyzed Keratin** | - | - | 0,1% | - | - | 0,1% | 0,2% | - | 0,1% | - |
| **Creatine** | - | 0,3% | - | - | - | - | - | - | - | 0,1% |
| **Panthenol** | 0,1% | 0,1% | 0,1% | 0,1% | 0,1% | 0,1% | - | 0,1% | 0,1% | 0,1% |
| **Citric Acid** | ad pH 5,5 | | | | | | | | | |
| **Perfumes, Dyes, Preservatives** | q.s. | | | | | | | | | |

| | **44a** | **44b** | **44c** | **44d** | **44e** | **44f** | **44g** | **44h** | **44i** | **44j** |
|---|---|---|---|---|---|---|---|---|---|---|
| **Wasser** | ad 100% | | | | | | | | | |
| **Diethylhexyl Sodium Sulfosuccinate** | 9,0% | 4,0% | 3,5% | 8,0% | 4,0% | 8,5% | 4,0% | 6,0% | 8,5% | 9,0% |
| **Sodium Laureth Sulfate** | 3,0% | 3,0% | - | 2,0% | 1,5% | - | 2,0% | 1,0% | 3,3% | - |
| **Sodium C14-16 Olefin Sulfonate** | - | - | - | 1,0% | - | 1,5% | - | - | - | - |
| **Coco-Betaine** | - | 3,0% | - | 0,5% | - | - | - | 1,0% | - | - |
| **Cocamidopropyl Betaine** | 3,0% | - | 3,0% | 2,5% | 1,0% | 2,5% | - | 1,0% | 3,0% | 2,7% |
| **Sodium Cocoamphopropionate** | - | - | - | - | 1,0% | 0,5% | 0,7% | - | - | - |
| **Disodium Cocoamphodiacetate** | - | - | - | 0,5% | - | - | 0,8% | - | - | 0,3% |
| **Disodium Laureth Sulfosuccinate** | - | - | 3,0% | - | 0,5% | - | 1,0% | 1,0% | - | 3,0% |
| **Coco-Glucoside** | - | - | - | - | - | - | 0,5% | - | 0,5% | 0,5% |
| **Sodium Cocoyl Glutamate** | - | - | - | - | - | - | 0,5% | 0,5% | 0,5% | - |
| **Sodium Cocoyl Glycinate** | - | - | - | - | 1,0% | 1,5% | - | 1,0% | 0,5% | - |
| **Sodium Lauroyl Methyl Isethionate** | - | - | - | 0,5% | - | 0,5% | - | - | 0,2% | - |
| **Sodium Cocoyl Sarcosinate** | - | - | - | - | 0,5% | - | 0,2% | 0,3% | - | - |
| **Laureth-5 Carboxylic Acid** | - | - | - | - | 0,2% | - | - | - | - | - |
| **Glycerin** | 1,0% | 0,3% | 0,5% | 0,5% | 0,3% | 0,5% | 0,5% | 0,5% | 0,3% | 1,0% |
| **PEG-40 Hydrogenated Castor Oil** | - | 0,3% | - | - | - | - | - | 0,3% | - | - |
| **Polyquaternium-7** | 0,2% | - | - | - | 0,1% | - | - | 0,2% | - | 0,3% |
| **Guar Hydroxypropyltrimonium Chloride** | - | - | 0,3% | - | 0,2% | - | 0,2% | 0,1% | - | - |
| **Dimethicone** | 1,2% | - | - | - | - | 0,5% | - | 0,2% | - | 0,3% |
| **Petrolatum** | - | - | 4% | 5% | - | 2% | 3% | - | - | 2% |
| **Helianthus Annuus Seed Oil** | 25% | 45% | 20% | 21% | 35% | 15% | - | 15% | - | 10% |
| **Ricinus Communis Seed Oil** | - | 10% | 10% | - | - | 5% | 15% | 10% | 15% | 2% |
| **Olea Europaea Fruit Oil** | - | - | 15% | - | 10% | - | 25% | 10% | 7% | 10% |
| **Acrylates / C10-30 Alkyl Acrylate Crosspolymer** | - | - | - | - | 0,3% | - | - | 0,3% | 0,2% | - |
| **Sodium Hydroxide, 25%** | - | - | - | - | 0,5% | - | - | 0,5% | 0,5% | - |
| **Cocamide MEA** | - | 0,8% | 1,0% | 0,5% | - | 0,2% | 0,6% | - | - | 0,3% |
| **Sodium Chloride** | 0,2% | - | - | - | - | 0,1% | - | - | 0,2% | 0,5% |
| **Propylene Glycol; PEG-55 Propylene Glycol Oleate** | - | - | - | - | 0,4% | - | - | - | - | - |
| **Sodium Hydroxypropyl Starch Phosphate** | - | - | - | - | - | 0,5% | - | - | - | 0,6% |
| **Hydroxypropyl Methylcellulose** | - | - | - | 0,3% | - | - | 0,3% | - | - | - |
| **Dicaprylylether** | - | - | 1,0% | - | - | - | 0,4% | - | - | - |
| **Cetearyl Alcohol** | - | - | - | - | - | - | 0,1% | - | - | 0,2% |
| **Benzophenone-4** | - | 0,1% | 0,2% | - | - | - | - | 0,2% | - | 0,1% |
| **Salicylic Acid** | - | 0,1% | - | - | - | - | 0,1% | - | - | - |
| **Octopirox** | 0,1% | - | - | 0,1% | - | - | - | - | - | 0,1% |
| **Caffeine** | - | - | 0,1% | - | 0,1% | - | - | 0,1% | - | 0,1% |
| **Benzyl Alcohol** | 0,1% | - | - | - | - | 0,1% | - | 0,1% | - | - |
| **Hydrolyzed Wheat Protein** | - | - | 0,1% | 0,1% | 0,1% | - | 0,2% | 0,1% | - | - |
| **Panthenol** | 0,1% | 0,1% | - | 0,1% | 0,1% | 0,1% | 0,1% | - | 0,1% | 0,1% |
| **Citric Acid** | ad pH 5,3 | | | | | | | | | |
| **Perfumes, Dyes, Preservatives** | q.s. | | | | | | | | | |

**Liste der eingesetzten Rohstoffe**

| **INCI** | **Handelsname, Firma** |
|---|---|
| Acrylates / C10-30 Alkyl Acrylate Crosspolymer | TEGO Carbomer 841 SER, Evonik Industries AG, 100% |
| Ammonium Laureth Sulfate | Sulfetal LA, Tschimmer & Schwarz, 28% |
| Amodimethicone | DC 949, Dow Corning, 100% |
| Argania Spinosa Oil (Argania Spinosa Kernel Oil) | Argan Oil, DSM Nutritional Products Ltd. |
| Benzophenone-4 | Uvinul MS 40, BASF |
| Benzyl Alcohol | Microcare BNA, THOR Personal Care SAS |
| Butyrospermum Parkii Butter Extract | Cosmosil 600, International Cosmetic Science Centre |
| Caffeine | Caffeine, Merck KGaA/EMD Chemicals, Inc. |
| Capryl/Capramidopropyl Betaine | TEGO Betaine 810, Evonik Industries AG, 38% |
| Caprylic/Capric Triglyceride | CAPRYLIC/CAPRIC TRIGLYCERIDE, Evonik Industries AG, 100% |
| Cellulose | Arbocel A300, J. Rettenmaier & Söhne |
| Cetearyl Alcohol | TEGO Alkanol 1618, Evonik Industries AG, 100% |
| Citric Acid | Citric Acid USP Granular, DSM Nutritional Products, Inc. |
| Cocamide DEA | REWOMID DC 212 S, Evonik Industries AG, 100% |
| Cocamide MEA | REWOMID D 212, Evonik Industries AG, 100% |
| Cocamidopropyl Betaine | TEGO Betain F 50, Evonik Industries AG, 38% |
| Coco-Glucoside | Plantacare 818 UP, BASF Cognis, 51% |
| Coco-Betaine | TEGO Betaine AB 1214, Evonik Industries AG, 31% |
| Creatine | TEGO Cosmo C 100, Evonik Industries AG, 100% |
| Dicaprylylether | Cetiol OE, BASF Cognis |
| Diethylhexyl Sodium Sulfosuccinate | TEGO Sulfosuccinate DO 75, Evonik Industries AG, 75% |
| Dimethicone | DC 200 Fluid 100 cSt, Dow Corning, 100% |
| Disodium Cocoamphodiacetate | REWOTERIC AM 2 C NM, Evonik Industries AG |
| Disodium Laureth Sulfosuccinate | REWOPOL SB FA 30 B, Evonik Industries AG, 40% |
| Disodium Lauryl Sulfosuccinate | REWOPOL SB F 12 P, Evonik Industries AG, 95% |
| Disodium PEG-4 Cocamido MIPA-Sulfosuccianate | REWOPOL SB Z U, Evonik Industries AG, 50% |
| Glycerin | Glycerol EP, vegetable, Spiga Nord, 99,7% |
| Glycine Soja (Soybean) Oil | Cropure Soybean, Croda Europe, Ltd. |
| Glycol Distearate | TEGIN G 1100, Evonik Industries AG, 100% |
| Helianthus Annuus (Sunflower) Seed Oil | Buttella Sonnenblumenöl, Brökelmann+ Co., Hamm, Deutschland. |
| Hydrogenated Castor Oil | Cutina HR Powder, BASF Personal Care and Nutrition Gmbh |
| Hydrolyzed Keratin | Kerasol, Croda, Inc. |
| Hydrolyzed Wheat Protein | Gluadin WLM, BASF Cognis |
| Hydroxypropyl Guar Hydroxypropyltrimonium Chloride | Jaguar C-162, Rhodia, 100% |
| Hydroxypropyl Methylcellulose | TEGOCEL HPM 50, Evonik Industries AG, 100% |
| Isostearamide MIPA | REWOMID IPP 240, Evonik Industries AG, 100% |
| Laureth-5 Carboxylic Acid | Marlowet 1072, Sasol Germany GmbH |
| Lauryl Glucoside | Plantacare 1200 UP, BASF Cognis, 50% |
| Octopirox | Octopirox, Clariant Intl. Ltd. |
| Olea Europaea (Olive) Fruit Oil | Cropure Olive, Croda Europe, Ltd. |
| Oleyl Erucate | TEGOSOFT OER, Evonik Industries AG, 100% |
| Palmitamidopropyltrimonium Chloride | VARISOFT PATC, Evonik Industries AG, 60% |
| Panthenol | D-Panthenol USP, BASF, 100% |
| PEG-200 Hydrogenated Glyceryl Palmate; PEG-7 Glyceryl Cocoate | REWODERM LI S 80, Evonik Industries AG, 100% |
| PEG-40 Hydrogenated Castor Oil | TAGAT CH 40, Evonik Industries AG, 100% |
| Persea Gratissima (Avocado) Oil | Cropure Avocado, Croda Europe, Ltd. |
| Petrolatum | Merkur 115, Sasol Wax GmbH |
| Polyglyceryl-4 Caprate | TEGOSOFT PC 41, Evonik Industries AG, 100% |
| Polyquaternium-10 | Polymer JR 400, Amerchol, 100% |
| Polyquaternium-7 | Merquat 550, Nalco, 100% |
| Propylene Glycol; PEG-55 Propylene Glycol Oleate | ANTIL 141 Liquid, Evonik Industries AG |
| Prunus Amygdalus Dulcis (Sweet Almond) Oil | Cropure Almond, Croda Europe, Ltd. |
| Ricinus Communis Seed Oil | Lipovol CO, Lipo Chemicals |
| Salicylic Acid | OriStar SCA, Orient Stars LLC |
| Silicone Quaternium-22 | ABIL T Quat 60, Evonik Industries AG, 65% |
| Simmondsia Chinensis (Jojoba) Seed Oil | AEC Jojoba Oil Refined, A & E Connock, Perfumery & Cosmetics Ltd. |
| Sodium C14-16 Olefin Sulfonate | Bioterge AS-40 AOS, Stepan, |
| Sodium Cocoamphoacetate | REWOTERIC AM C, Evonik Industries AG, 32% |
| Sodium Cocoamphopropionate | REWOTERIC AM KSF 40, Evonik Industries AG, 40% |
| Sodium Cocoyl Glutamate | Plantapon ACG HC, BASF Cognis |
| Sodium Cocoyl Glycinate | Hostapon SG, Clariant; Amilite GCS-11, Ajinomoto |
| Sodium Cocoyl Sarcosinate | Crodasinic CS, Croda |
| Sodium/Disodium Cocoyl Glutamate | PERLASTAN SC 25 NKW, Schill&Seilacher, 25%-ig, |
| Sodium Hydroxide | Unichem SOHYD, Universal Preserv-A-Chem, Inc. |
| Sodium Hydroxypropyl Starch Phosphate | Pure-Gel, Grain Processing Corporation |
| Sodium Laureth Sulfate | Texapon NSO, BASF Cognis, 28% |
| Sodium Lauroyl Methyl Isethionate | Iselux, Innospec Active Chemicals |
| Sodium Lauryl Sulfate | Texapon LS 35, BASF Cognis, 30% |
| Stearic Acid | Pristerene 4922, Croda Europe, Ltd. |
| Tetrasodium EDTA | Versene 100, The Dow Chemical Company |
| Xanthan Gum | Keltrol CG-SFT, CP Kelco, 100% |
| Zinc Pyrithione | Microcare ZP, Thor Personal Care SAS |

## Patentansprüche

1. Zusammensetzung enthaltend
A) 20 Gewichtsteile bis 67 Gewichtsteile, bevorzugt 25 Gewichtsteile bis 57 Gewichtsteile, besonders bevorzugt 33 Gewichtsteile bis 47 Gewichtsteile, mindestens einer Substanz ausgewählt aus Dialkylsulfosuccinaten und Dialkylmethylsulfosuccinaten,
B) 15 Gewichtsteile bis 40 Gewichtsteile, bevorzugt 20 Gewichtsteile bis 32 Gewichtsteile, besonders bevorzugt 23 Gewichtsteile bis 28 Gewichtsteile, mindestens eines amphoteren Tensids, und
C) 15 Gewichtsteile bis 40 Gewichtsteile, bevorzugt 20 Gewichtsteile bis 32 Gewichtsteile, besonders bevorzugt 23 Gewichtsteile bis 28 Gewichtsteile, mindestens eines anionischen Tensids.

2. Zusammensetzung gemäß Anspruch 1, **dadurch gekennzeichnet, dass** das enthaltene Dialkylsulfosuccinat und/oder enthaltene Dialkylmethylsulfosuccinat ausgewählt sind aus der Gruppe bestehend aus Dialkylsulfosuccinaten und/oder Dialkylmethylsulfosuccinaten, bei denen die Alkylreste jeweils 4 bis 24, bevorzugt 6 bis 18, besonders bevorzugt 6 bis 14, Kohlenstoffatome aufweisen.

3. Zusammensetzung gemäß mindestens einem der vorherigen Ansprüche, **dadurch gekennzeichnet, dass** das enthaltene amphotere Tensid ausgewählt ist aus der Gruppe umfassend, bevorzugt bestehend aus Alkylbetaine, Alkylamidoalkylbetaine, Alkylamphoacetate, Alkylamphodiacetate, Alkylamphopropionate, Alkylamphodipropionate, Alkylsultaine, Alkylhydroxysultaine, Alkylaminoxide, Alkylamphoglycinate, Alkyliminodiacetate, Alkyliminodipropionate, Alkylamphopropylsulfonate sowie Alkali-, Erdalkali- oder Ammoniumsalzen von Alkylamphocarboxyglycinaten und Alkylamphocarboxypropionaten.

4. Zusammensetzung gemäß mindestens einem der vorherigen Ansprüche, **dadurch gekennzeichnet, dass** das enthaltene anionische Tensid ausgewählt ist aus der Gruppe umfassend, bevorzugt bestehend aus, Alkylsulfaten, Alkylethersulfaten, gegebenenfalls alkoxylierte Sulfosuccinaten, gegebenenfalls alkoxylierte Methylsulfosuccinaten, gegebenenfalls alkoxylierte Sulfonaten, gegebenenfalls alkoxylierte Glycinaten, gegebenenfalls alkoxylierte Glutamaten, gegebenenfalls alkoxylierte Isethionaten, gegebenenfalls alkoxylierte Carboxylaten, gegebenenfalls alkoxylierte Anisate, gegebenenfalls alkoxylierte Levulinate, gegebenenfalls alkoxylierte Tartrate, gegebenenfalls alkoxylierte Lactylate, gegebenenfalls alkoxylierte Taurate, gegebenenfalls alkoxylierte Alaninate, gegebenenfalls alkoxylierte Phosphate, gegebenenfalls alkoxylierte Sulfoacetate, gegebenenfalls alkoxylierte Sulfosuccinamate, gegebenenfalls alkoxylierte Sarcosinaten und gegebenenfalls alkoxylierte Phosphonaten.

5. Zusammensetzung gemäß mindestens einem der vorherigen Ansprüche, **dadurch gekennzeichnet, dass** sie
D) 0 Gewichtsteile bis 15 Gewichtsteile, bevorzugt 5 Gewichtsteile bis 12 Gewichtsteile, besonders bevorzugt 7 Gewichtsteile bis 10 Gewichtsteile, mindestens eines hydrophoben Verdickers
enthält.

6. Zusammensetzung gemäß mindestens einem der vorherigen Ansprüche, **dadurch gekennzeichnet, dass** der enthaltene hydrophobe Verdicker ausgewählt ist aus der Gruppe enthaltend, bevorzugt bestehend aus, Umsetzungsprodukte mindestens einer Fettsäure und/oder mindestens eines Fettsäureesters, wie zum Beispiel Fettsäureglycerinester, Fettsäuremethylester und Fettsäureethylester, mit mindestens einem primären oder sekundären Amin, wobei das Amin bevorzugt zusätzlich noch eine oder mehrere andere funktionale Gruppen wie beispielsweise Alkohole enthält, und
kationische Tenside, bei welchen bei einem pH-Wert von 7 und 20 °C mindestens 90 mol-% der Moleküle mindestens eine positiv geladene Gruppe aufweisen und bevorzugt keinen isolelektischen Punkt von pH_{IEP}=2-12 bei 25°C aufweisen, wobei in einer wässrigen 10 millimolaren Kaliumchloridlösung als Hintergrundelektrolyt gemessen wird.

7. Zusammensetzung gemäß mindestens einem der vorherigen Ansprüche, **dadurch gekennzeichnet, dass** sie ein Lösungsmittel enthält.

8. Strukturierte Formulierung enthaltend
eine Zusammensetzung gemäß mindestens einem der vorherigen Ansprüche und
E) mindestens ein kosmetisches Öl in einer Menge von 11 Gew.-% bis 74 Gew.-%, bevorzugt 16 Gew.-% bis 60 Gew.-%, besonders bevorzugt 21 Gew.-% bis 55 Gew.-%,
wobei sich die Gewichtsprozente auf die Gesamtformulierung beziehen.

9. Strukturierte Formulierung gemäß Anspruch 8 in Summe enthaltend die Komponenten A) bis D) in einer Menge von 5 Gew.-% bis 35 Gew.-%, bevorzugt 8 Gew.-% bis 25 Gew.-%, besonders bevorzugt 11 Gew.-% bis 20 Gew.-%, wobei sich die Gewichtsprozente auf die Gesamtformulierung beziehen.

10. Strukturierte Formulierung gemäß Anspruch 8 oder 9 aufweisend eine Fließgrenze von größer 0,001 Pa.

11. Strukturierte Formulierung gemäß mindestens einem der Ansprüche 8 bis 10, **dadurch gekennzeichnet, dass** das kosmetische Öl ausgewählt ist aus der Gruppe Silikonöle, funktionalisierte Silikone, Mineralöle, Fettsäureester, natürliche Öle, wie pflanzliche Öle, tierische Öle sowie deren Mischungen.

12. Strukturierte Formulierung gemäß mindestens einem der Ansprüche 8 bis 11, **dadurch gekennzeichnet, dass** sie eine kosmetische Pflege- und Reinigungsformulierungen, insbesondere für Haut und Hautanhangsgebilde, ist.

13. Verfahren zur Herstellung von strukturierten Formulierungen umfassend die Schritte
1) Bereitstellen einer Zusammensetzung nach mindestens einem der Ansprüche 1 bis 7
2) Mischen der erfindungsgemäßen Zusammensetzung mit
E) mindestens einem kosmetischen Öl unter Erhalt einer strukturierten Formulierung enthaltend das Öl in einer Menge von 11 Gew.-% bis 74 Gew.-%, bevorzugt 16 Gew.-% bis 60 Gew.-%, besonders bevorzugt 21 Gew.-% bis 55 Gew.-%, wobei sich die Gewichtsprozente auf die Gesamtformulierung beziehen.

14. Verfahren zur Herstellung von strukturierten Formulierungen umfassend die Schritte
a) Bereitstellen einer Zusammensetzung enthaltend
B) 15 Gewichtsteile bis 40 Gewichtsteile, bevorzugt 22 Gewichtsteile bis 32 Gewichtsteile, besonders bevorzugt 23 Gewichtsteile bis 28 Gewichtsteile, mindestens eines amphoteren Tensids,
C) 15 Gewichtsteile bis 40 Gewichtsteile, bevorzugt 22 Gewichtsteile bis 32 Gewichtsteile, besonders bevorzugt 23 Gewichtsteile bis 28 Gewichtsteile, mindestens eines anionischen Tensids, und
D) 0 Gewichtsteile bis 15 Gewichtsteile, bevorzugt 5 Gewichtsteile bis 12 Gewichtsteile, besonders bevorzugt 7 Gewichtsteile bis 10 Gewichtsteile, mindestens eines hydrophoben Verdickers,
b) Hinzufügen und Mischen der Komponenten A) und E) zu der Zusammensetzung aus Verfahrensschritt a)
A) 20 Gewichtsteile bis 67 Gewichtsteile, bevorzugt 25 Gewichtsteile bis 57 Gewichtsteile, besonders bevorzugt 33 Gewichtsteile bis 47 Gewichtsteile, mindestens einer Substanz ausgewählt aus Dialkylsulfosuccinaten und Dialkylmethylsulfosuccinaten, und
E) mindestens ein kosmetisches Öl in einer Menge von 11 Gew.-% bis 74 Gew.-%, bevorzugt 16 Gew.-% bis 60 Gew.-%, besonders bevorzugt 21 Gew.-% bis 55 Gew.-%, wobei sich die Gewichtsprozente für das kosmetische Öl auf die zu erhaltende Gesamtformulierung beziehen, und
wobei die Summe der Gewichtsteile der Komponenten A) bis D) 100 Gewichtsteile ergeben.

## Claims

1. Composition comprising
A) 20 parts by weight to 67 parts by weight, preferably 25 parts by weight to 57 parts by weight, particularly preferably 33 parts by weight to 47 parts by weight, of at least one substance selected from dialkyl sulfosuccinates and dialkyl methylsulfosuccinates,
B) 15 parts by weight to 40 parts by weight, preferably 20 parts by weight to 32 parts by weight, particularly preferably 23 parts by weight to 28 parts by weight, of at least one amphoteric surfactant, and
C) 15 parts by weight to 40 parts by weight, preferably 20 parts by weight to 32 parts by weight, particularly preferably 23 parts by weight to 28 parts by weight, of at least one anionic surfactant.

2. Composition according to Claim 1, **characterized in that** the dialkyl sulfosuccinate present and/or dialkyl methylsulfosuccinate present are selected from the group consisting of dialkyl sulfosuccinates and/or dialkyl methylsulfosuccinates in which the alkyl radicals each have 4 to 24, preferably 6 to 18, particularly preferably 6 to 14 carbon atoms.

3. Composition according to at least one of the preceding claims, **characterized in that** the amphoteric surfactant present is selected from the group comprising, preferably consisting of, alkylbetaines, alkylamidoalkylbetaines, alkyl amphoacetates, alkyl amphodiacetates, alkyl amphopropionates, alkyl amphodipropionates, alkylsultaines, alkylhydroxysultaines, alkylamine oxides, alkyl amphoglycinates, alkyl iminodiacetates, alkyl iminodipropionates, alkyl amphopropylsulfonates, and also alkali metal, alkaline earth metal or ammonium salts of alkyl amphocarboxyglycinates and alkyl amphocarboxypropionates.

4. Composition according to at least one of the preceding claims, **characterized in that** the anionic surfactant present is selected from the group comprising, preferably consisting of, alkyl sulfates, alkyl ether sulfates, optionally alkoxylated sulfosuccinates, optionally alkoxylated methylsulfosuccinates, optionally alkoxylated sulfonates, optionally alkoxylated glycinates, optionally alkoxylated glutamates, optionally alkoxylated isethionates, optionally alkoxylated carboxylates, optionally alkoxylated anisates, optionally alkoxylated levulinates, optionally alkoxylated tartrates, optionally alkoxylated lactylates, optionally alkoxylated taurates, optionally alkoxylated alaninates, optionally alkoxylated phosphates, optionally alkoxylated sulfoacetates, optionally alkoxylated sulfosuccinates, optionally alkoxylated sarcosinates and optionally alkoxylated phosphonates.

5. Composition according to at least one of the preceding claims, **characterized in that** it comprises
D) 0 parts by weight to 15 parts by weight, preferably 5 parts by weight to 12 parts by weight, particularly preferably 7 parts by weight to 10 parts by weight, of at least one hydrophobic thickener.

6. Composition according to at least one of the preceding claims, **characterized in that** the hydrophobic thickener present is selected from the group comprising, preferably consisting of, reaction products of at least one fatty acid and/or at least one fatty acid ester, such as for example fatty acid glycerol esters, fatty acid methyl esters and fatty acid ethyl esters, with at least one primary or secondary amine, where the amine preferably additionally also comprises one or more other functional groups such as for example alcohols, and
cationic surfactants in which, at a pH of 7 and 20°C, at least 90 mol% of the molecules have at least one positively charged group and preferably have no isoelectric point of pH_{IEP}= 2-12 at 25°C, measurement being carried out in an aqueous 10 millimolar potassium chloride solution as background electrolyte.

7. Composition according to at least one of the preceding claims, **characterized in that** it comprises a solvent.

8. Structured formulation comprising
a composition according to at least one of the preceding claims and
E) at least one cosmetic oil in an amount of from 11% by weight to 74% by weight, preferably 16% by weight to 60% by weight, particularly preferably 21% by weight to 55% by weight,
where the percentages by weight refer to the total formulation.

9. Structured formulation according to Claim 8, in total comprising the components A) to D) in an amount of from 5% by weight to 35% by weight, preferably 8% by weight to 25% by weight, particularly preferably 11% by weight to 20% by weight, where the percentages by weight refer to the total formulation.

10. Structured formulation according to Claim 8 or 9, having a yield point of greater than 0.001 Pa.

11. Structured formulation according to at least one of Claims 8 to 10, **characterized in that** the cosmetic oil is selected from the group silicone oils, functionalized silicones, mineral oils, fatty acid esters, natural oils, such as vegetable oils, animal oils, and mixtures thereof.

12. Structured formulation according to at least one of Claims 8 to 11, **characterized in that** it is a cosmetic care and cleansing formulation, in particular for skin and skin appendages.

13. Process for preparing structured formulations, comprising the steps
1) Provision of a composition according to at least one of Claims 1 to 7
2) mixing of the composition according to the invention with
E) at least one cosmetic oil to give a structured formulation comprising the oil in an amount of from 11% by weight to 74% by weight, preferably 16% by weight to 60% by weight, particularly preferably 21% by weight to 55% by weight, where the percentages by weight refer to the total formulation.

14. Process for preparing structured formulations, comprising the steps
a) provision of a composition comprising
B) 15 parts by weight to 40 parts by weight, preferably 22 parts by weight to 32 parts by weight, particularly preferably 23 parts by weight to 28 parts by weight, of at least one amphoteric surfactant,
C) 15 parts by weight to 40 parts by weight, preferably 22 parts by weight to 32 parts by weight, particularly preferably 23 parts by weight to 28 parts by weight, of at least one anionic surfactant, and
D) 0 parts by weight to 15 parts by weight, preferably 5 parts by weight to 12 parts by weight, particularly preferably 7 parts by weight to 10 parts by weight, of at least one hydrophobic thickener,
b) adding and mixing the components A) and E) to the composition from process step a)
A) 20 parts by weight to 67 parts by weight, preferably 25 parts by weight to 57 parts by weight, particularly preferably 33 parts by weight to 47 parts by weight, of at least one substance selected from dialkyl sulfosuccinates and dialkyl methylsulfosuccinates, and
E) at least one cosmetic oil in an amount of from 11% by weight to 74% by weight, preferably 16% by weight to 60% by weight, particularly preferably 21% by weight to 55% by weight, where the percentages by weight for the cosmetic oil refer to the total formulation to be obtained, and
where the sum of the parts by weight of components A) to D) gives 100 parts by weight.

## Revendications

1. Composition contenant
A) 20 parties en poids à 67 parties en poids, de préférence 25 parties en poids à 57 parties en poids, de manière particulièrement préférée 33 parties en poids à 47 parties en poids, d'au moins une substance choisie parmi les sulfosuccinates de dialkyle et les sulfosuccinates de dialkylméthyle,
B) 15 parties en poids à 40 parties en poids, de préférence 20 parties en poids à 32 parties en poids, de manière particulièrement préférée 23 parties en poids à 28 parties en poids, d'au moins un tensioactif amphotère, et
C) 15 parties en poids à 40 parties en poids, de préférence 20 parties en poids à 32 parties en poids, de manière particulièrement préférée 23 parties en poids à 28 parties en poids, d'au moins un tensioactif anionique.

2. Composition selon la revendication 1, **caractérisée en ce que** le sulfosuccinate de dialkyle contenu et/ou le sulfosuccinate de dialkylméthyle contenu sont choisis dans le groupe constitué par les sulfosuccinates de dialkyle et/ou les sulfosuccinates de dialkylméthyle dans lesquels les radicaux alkyle comprennent chacun 4 à 24, de préférence 6 à 18, de manière particulièrement préférée 6 à 14, atomes de carbone.

3. Composition selon au moins l'une quelconque des revendications précédentes, **caractérisée en ce que** le tensioactif amphotère contenu est choisi dans le groupe comprenant, de préférence constitué par, les alkylbétaïnes, les alkylamidoalkylbétaïnes, les alkylamphoacétates, les alkylamphodiacétates, les alkylamphopropionates, les alkylamphodipropionates, les alkylsultaïnes, les alkylhydroxysultaïnes, les alkylaminoxydes, les alkylamphoglycinates, les alkyliminodiacétates, les alkyliminodipropionates, les alkylamphopropylsulfonates, ainsi que les sels alcalins, alcalino-terreux ou d'ammonium d'alkylamphocarboxyglycinates et d'alkylamphocarboxypropionates.

4. Composition selon au moins l'une quelconque des revendications précédentes, **caractérisée en ce que** le tensioactif anionique contenu est choisi dans le groupe comprenant, de préférence constitué par, les alkylsulfates, les alkyléther-sulfates, les sulfosuccinates éventuellement alcoxylés, les méthylsulfosuccinates éventuellement alcoxylés, les sulfonates éventuellement alcoxylés, les glycinates éventuellement alcoxylés, les glutamates éventuellement alcoxylés, les iséthionates éventuellement alcoxylés, les carboxylates éventuellement alcoxylés, les anisates éventuellement alcoxylés, les lévulinates éventuellement alcoxylés, les tartrates éventuellement alcoxylés, les lactylates éventuellement alcoxylés, les taurates éventuellement alcoxylés, les alaninates éventuellement alcoxylés, les phosphates éventuellement alcoxylés, les sulfoacétates éventuellement alcoxylés, les sulfosuccinamates éventuellement alcoxylés, les sarcosinates éventuellement alcoxylés et les phosphonates éventuellement alcoxylés.

5. Composition selon au moins l'une quelconque des revendications précédentes, **caractérisée en ce qu'**elle contient
D) 0 partie en poids à 15 parties en poids, de préférence 5 parties en poids à 12 parties en poids, de manière particulièrement préférée 7 parties en poids à 10 parties en poids, d'au moins un épaississant hydrophobe.

6. Composition selon au moins l'une quelconque des revendications précédentes, **caractérisée en ce que** l'épaississant hydrophobe contenu est choisi dans le groupe contenant, de préférence constitué par,
les produits de réaction d'au moins un acide gras et/ou d'au moins un ester d'acide gras, tel que par exemple un ester de glycérine d'acide gras, un ester méthylique d'acide gras et un ester éthylique d'acide gras, avec au moins une amine primaire ou secondaire, l'amine contenant de préférence en outre un ou plusieurs autres groupes fonctionnels, tels que par exemple des alcools, et
les tensioactifs cationiques, dans lesquels à un pH de 7 et à 20 °C, au moins 90 % en moles des molécules comprennent au moins un groupe chargé positivement, et ne présentent de préférence pas de point isoélectrique de pH_{IEP} = 2 à 12 à 25 °C, la mesure étant effectuée dans une solution aqueuse de chlorure de potassium 10 millimolaire en tant qu'électrolyte d'arrière-plan.

7. Composition selon au moins l'une quelconque des revendications précédentes, **caractérisée en ce qu'**elle contient un solvant.

8. Formulation structurée, contenant
une composition selon au moins l'une quelconque des revendications précédentes, et
E) au moins une huile cosmétique en une quantité de 11 % en poids à 74 % en poids, de préférence de 16 % en poids à 60 % en poids, de manière particulièrement préférée de 21 % en poids à 55 % en poids,
les pourcentages en poids se rapportant à la formulation totale.

9. Formulation structurée selon la revendication 8, contenant au total les composants A) à D) en une quantité de 5 % en poids à 35 % en poids, de préférence de 8 % en poids à 25 % en poids, de manière particulièrement préférée de 11 % en poids à 20 % en poids, les pourcentages en poids se rapportant à la formulation totale.

10. Formulation structurée selon la revendication 8 ou 9, présentant une limite d'écoulement de plus de 0,001 Pa.

11. Formulation structurée selon au moins l'une quelconque des revendications 8 à 10, **caractérisée en ce que** l'huile cosmétique est choisie dans le groupe constitué par les huiles de silicone, les silicones fonctionnalisées, les huiles minérales, les esters d'acides gras, les huiles naturelles, telles que les huiles végétales, les huiles animales et leurs mélanges.

12. Formulation structurée selon au moins l'une quelconque des revendications 8 à 11, **caractérisée en ce qu'**il s'agit d'une formulation cosmétique de soin et de nettoyage, notamment pour la peau et les annexes de la peau.

13. Procédé de fabrication de formulations structurées comprenant les étapes suivantes :
1) la préparation d'une composition selon au moins l'une quelconque des revendications 1 à 7,
2) le mélange de la composition selon l'invention avec
E) au moins une huile cosmétique pour obtenir une formulation structurée contenant l'huile en une quantité de 11 % en poids à 74 % en poids, de préférence de 16 % en poids à 60 % en poids, de manière particulièrement préférée de 21 % en poids à 55 % en poids, les pourcentages en poids se rapportant à la formulation totale.

14. Procédé de fabrication de formulations structurées, comprenant les étapes suivantes :
a) la préparation d'une composition contenant
B) 15 parties en poids à 40 parties en poids, de préférence 22 parties en poids à 32 parties en poids, de manière particulièrement préférée 23 parties en poids à 28 parties en poids, d'au moins un tensioactif amphotère,
C) 15 parties en poids à 40 parties en poids, de préférence 22 parties en poids à 32 parties en poids, de manière particulièrement préférée 23 parties en poids à 28 parties en poids, d'au moins un tensioactif anionique, et
D) 0 partie en poids à 15 parties en poids, de préférence 5 parties en poids à 12 parties en poids, de manière particulièrement préférée 7 parties en poids à 10 parties en poids, d'au moins un épaississant hydrophobe,
b) l'ajout et le mélange des composants A) et E) à la composition de l'étape de procédé a),
A) 20 parties en poids à 67 parties en poids, de préférence 25 parties en poids à 57 parties en poids, de manière particulièrement préférée 33 parties en poids à 47 parties en poids, d'au moins une substance choisie parmi les sulfosuccinates de dialkyle et les sulfosuccinates de dialkylméthyle, et
E) au moins une huile cosmétique en une quantité de 11 % en poids à 74 % en poids, de préférence de 16 % en poids à 60 % en poids, de manière particulièrement préférée de 21 % en poids à 55 % en poids, les pourcentages en poids pour l'huile cosmétique se rapportant à la formulation totale obtenue, et
la somme des parties en poids des composants A) à D) étant de 100 parties en poids.
